# EUROPÄISCHE PATENTANMELDUNG

(11) **EP 3 170 510 A1**
(43) Veröffentlichungstag der Anmeldung: **24.05.2017**
(21) Anmeldenummer: 16002678.7
(22) Anmeldetag: 17.12.2010
(51) Int. Cl.: A61K 39/36, C07K 14/415

(54) **VARIANTEN DER GRUPPE 5-ALLERGENE DER SÜSSGRÄSER MIT REDUZIERTER ALLERGENITÄT DURCH MUTAGENESE VON PROLINRESTEN**

(30) Priorität: 14.01.2010 EP 10000296
(62) Teilanmeldung aus: 10795625.2
(71) Anmelder: Merck Patent GmbH, 64293 Darmstadt (DE)
(72) Erfinder: Wald, Martin, 20253 Hamburg (DE); Nandy, Andreas, 22175 Hamburg (DE); Fiebig, Helmut, 21493 Schwarzenbek (DE); Weber, Bernhard, 21031 Hamburg (DE); Kahlert, Helga, 22041 Hamburg (DE); Reese, Gerald, 63225 Langen (DE); Cromwell, Oliver, 23701 Suesel-Fassensdorf (DE)

(57) **Zusammenfassung**

Die vorliegende Erfindung betrifft die Herstellung und Verwendung von rekombinanten Varianten der Gruppe-5-Allergene der Poaceae (Süßgräser), welche durch eine gegenüber den bekannten Wildtyp-Allergenen verringerte IgE-Reaktivität und gleichzeitig eine weitgehend erhaltene Reaktivität mit T-Lymphozyten gekennzeichnet sind.

## Beschreibung

### Gebiet der Erfindung

Die vorliegende Erfindung betrifft die Herstellung und Verwendung von rekombinanten Varianten der Gruppe 5-Allergene der Poaceae (Süßgräser), welche durch eine gegenüber den bekannten Wildtyp-Allergenen verringerte IgE-Reaktivität und gleichzeitig eine weitgehend erhaltene Reaktivität mit T-Lymphozyten gekennzeichnet sind.

Die verringerte IgE-Reaktivität wird hauptsächlich durch die Substitution oder Deletion bestimmter Prolinreste, die bei Gruppe 5-Allergenen stark konserviert sind, erreicht. Zudem wurde die Aminosäure Prolin an Position 211 (Phl p 5.0109 Nummerierung) als Schlüsselposition für die gezielte Beinflussung des Löslichkeitsverhalten der Varianten in wässrigen Formulierungen erkannt.

Diese hypoallergenen Allergenvarianten können zur spezifischen Immuntherapie (Hyposensibilisierung) von Patienten mit Gräserpollenallergie oder zur präventiven Behandlung zur Vermeidung der Ausprägung von Gräserpollenallergien eingesetzt werden.

Eine bevorzugte Ausführungsform der Erfindung betrifft Varianten des Hauptallergens Phl p 5 aus dem Pollen des Wiesenlieschgrases (*Phleum pratense*).

### Hintergrund der Erfindung

Allergien vom Typ 1 haben weltweite Bedeutung. Bis zu 20% der Bevölkerung in industrialisierten Ländern leiden unter Beschwerden wie allergischer Rhinits, Konjunktivitis oder Bronchialasthma.

Diese Allergien werden von Quellen unterschiedlicher Herkunft wie Bäumen und Gräser (Pollen), Pilzen (Sporen), Milben (Kot), Katzen oder Hunden hervorgerufen. Die Allergenquellen werden direkt in die Luft abgegeben (Pollen, Sporen) oder können gebunden an Dieselrußpartikel (Pollen) oder Hausstaub (Milbenkot, Hautpartikel, Haare) in die Luft gelangen. Da sie sich die Allergie auslösenden Substanzen in der Luft befinden, spricht man auch von Aeroallergenen.

Bei den Typ 1-Allergie auslösenden Substanzen handelt es sich um Proteine, Glykoproteine oder Polypeptide. Diese Allergene reagieren nach Aufnahme über Schleimhäute mit den bei sensibilisierten Personen an der Oberfläche von Mastzellen gebundenen IgE-Molekülen. Werden diese IgE-Moleküle durch ein Allergen miteinander vernetzt, führt dies zur Ausschüttung von Mediatoren (z. B. Histamin, Prostaglandine) und Zytokinen durch die Effektorzelle und damit zu den entsprechenden allergischen Symptomen.

Bis zu 40% der Typ 1-Allergiker zeigen spezifische IgE-Reaktivität mit Pollenextrakten von Süßgräsern (Burney et al., 1997, J. Allergy Clin. Immunol. 99:314-322; D'Amato et al., 1998, Allergy 53: 567-578; Freidhoff et al., 1986, J. Allergy Clin Immunology, 78, 1190-2002). Die Familie der Süßgräser *(Poaceae)* umfasst über 10000 Spezies, von denen bisher weit über 20 als Auslöser von allergischen Symptomen bekannt sind (Andersson & Lidholm, 2003, Int. Arch. Allergy Immunol. 130:87-107; Esch, 2008, Allergens and Allergen Immunotherapy, Clinical Allergy and Immunology Series, 107-126).

Die meisten der Allergie auslösenden Süßgräser gehören zur Unterfamilie der *Pooideae.* Neben den als Wildformen vorkommenden Gräserarten wie beispielsweise *Holcus Ianatus* (Wolliges Honiggras), *Phalaris aquatica* (Knolliges Glanzgras), Anthoxanthum odoratum (Gewöhnliches Ruchgras), *Dactylis glomerata* (Gewöhnliches Knäuelgras), *Festuca pratensis* (Wiesenschwingel), *Poa pratensis* (Wiesenrispengras) oder *Lolium perenne* (Deutsches Weidelgras) sind auch kultivierte Cerealien wie *Triticum aestivum* (Weizen), *Secale cereale* (Roggen) und *Hordeum vulgare* (Gerste) bekannte Mitglieder dieser Unterfamilie.

Eine der hinsichtlich ihrer Allergene am besten untersuchten Spezies der *Pooideae* ist das Wiesenlieschgras (*Phleum pratense*), welches weltweit als Wildpflanze verbreitet ist und als Weidepflanze und winterhartes Futtergras auch eine wirtschaftliche Rolle spielt.

In Abhängigkeit von der relativen Häufigkeit in einer Population, mit der die einzelnen Allergenmoleküle mit den IgE-Antikörpern von Allergikern reagieren, wird zwischen Major- und Minorallergenen unterschieden.

Sechs Allergene des Wiesenlieschgrases können als Majorallergene angesehen werden: Phl p 1 (Petersen et al., 1993, J. Allergy Clin. Immunol. 92: 789-796), Phl p 5 (Matthiesen und Löwenstein, 1991, Clin. Exp. Allergy 21: 297-307; Petersen et al., 1992, Int. Arch. Allergy Immunol. 98: 105-109), Phl p 6 (Petersen et al., 1995, Int. Arch. Allergy Immunol. 108, 49-54), Phl p 2/3 (Dolecek et al., 1993, FEBS 335 (3): 299-304), Phl p 4 (Haavik et al., 1985, Int. Arch. Allergy Appl. Immunol. 78: 260-268; Valenta et al., 1992, Int. Arch. Allergy Immunol. 97: 287-294; Nandy et al., Biochem. Biophys. Res. Commun., 2005, 337(2): 563-70) und Phl p 13 (Suck et al., 2000, Clin. Exp. Allergy 30: 1395-1402).

Die dominierenden Majorallergene des Wiesenlieschgrases sind Phl p 1 und Phl p 5 (Andersson & Lidholm, 2003, Int. Arch. Allergy Immunol. 130:87-107), wobei Phl p 5 in den zwei hinsichtlich ihrer Molekularmasse differenten Formen 5a und 5b, die von unabhängigen Genen kodiert werden, vorkommt. Folgend der offiziellen Nomenklatur für Allergene wird Phl p 5a als Phl p 5.01 und Phl p 5b als Phl p 5.02 benannt (WHO/IUIS Allergen Nomenclature Subcommittee, www.allergen.org). Die Aminosäuresequenzen sowohl von Phl p 5a als auch von Phl p 5b konnten von den klonierten cDNA-Sequenzen abgeleitet werden. Von beiden Isoformen wurden natürliche Varianten identifiziert, die sich durch Punktmutationen voneinander unterscheiden und unterschiedlichen allelen Formen entsprechen (Vrtala et al., 1993, J. Immunol., 151: 4773-4781; Gelhar et al., 1997, Eur. J. Biochem., 247: 217-23). Diese Varianten werden in der WHO/IUIS Datenbank als Phl p 5.01 xx und Phl p 5.02xx geführt.

Das natürliche Phl p 5a (nPhl p 5a) ist ein Protein von ca. 32 kDa und reagiert mit IgE-Antikörpern von 85-90% der Gräserpollenallergiker (Rossi et al., 2000, Allergy Int., 49: 93-97).

Die Pollen der verwandten Süßgräserarten der Familie *Poaceae* und insbesondere der Unterfamilie *Pooideae,* wie *Lolium perenne* oder Poa *pratensis* enthalten Allergene, die dem Phl p 5 homolog sind und zusammen als Gruppe 5-Allergene bezeichnet werden. Die hohe strukturelle Homologie dieser Gruppe 5-Allergene bedingt eine entsprechend hohe Kreuzreaktivität der Moleküle mit IgE-Antikörpern (Lorenz et al., 2009, Int. Arch. Immunol. 148:1-17). Letztlich kann aufgrund dieser Kreuzreaktivität eine Sensibilisierung durch eine Gräserart ausreichen, um eine allergische Reaktion durch andere verwandte Gräser auszulösen.

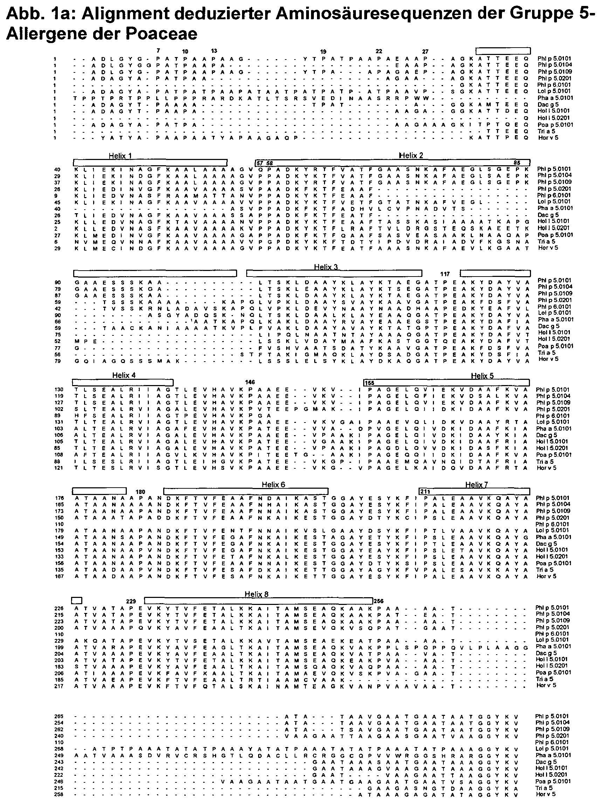

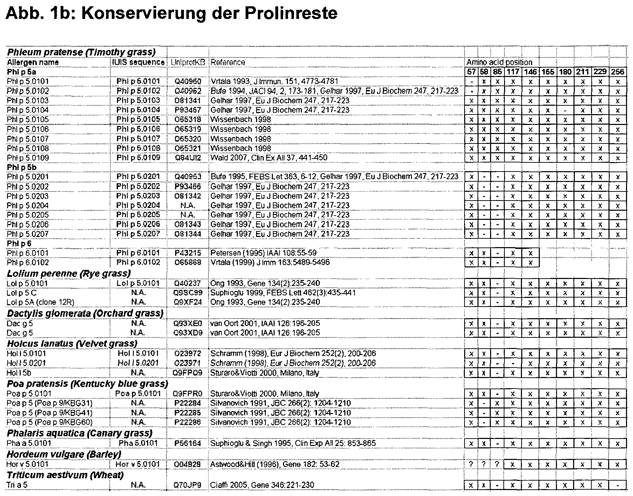

Die hohe Kreuzreaktivität der Gruppe 5-Allergene basiert letztlich auf einer ähnlichen Primärsequenz der homologen Allergene. Dies zeigt ein Aminosäure-Sequenzvergleich von Gruppe 5-Allergenen von ausgewählten Spezies der *Pooideae* (Abb. 1).

Neben der Kreuzreaktivität der Gruppe 5-Allergene untereinander ist auch eine Kreuzreaktivität des Phl p 5 mit einem anderen Majorallergen des Wiesenlieschgrases bekannt (Løwenstein, 1978, Allergy 33: 30-41; Petersen et al., 1995, Int. Arch. Allergy Immunol. 108: 55-59; Blume et al., 2004, Proteomics 4: 1366-71). Die Polypeptidkette des Allergens Phl p 6 zeigt eine große Ähnlichkeit mit der N-terminalen Hälfte der verschiedenen Phl p 5-Sequenzen (Abb. 1). Es wird vermutet, dass die Allergene auf ein gemeinsames Ursprungsgen zurückzuführen sind. Die Ähnlichkeit zwischen den Allergenen der beiden Gruppen bewirkt, dass ein Teil der Phl p 5-reaktiven IgE-Antikörper auch an Phl p 6 bindet (Petersen et al., 1995, Int. Arch. Allergy Immunol. 108: 49-54; Andersson & Lidholm, 2003, Int. Arch. Allergy Immunol. 130:87-107).

Die 3D-Struktur vieler Allergene ist in der Vergangenheit durch NMR-Spektroskopie oder Röntgenstrukturanalyse aufgeklärt worden und diente unter anderem als Grundlage zur Lokalisierung von IgE-bindenden Epitopen auf der Proteinoberfläche. Im Fall der Gruppe 5-Allergene der Gräserpollen ist es bisher nicht gelungen ein Modell zu generieren, das die gesamte Polypeptidkette umfasst (Rajashankar et al., 2002, Acta Cryst. D58:1175-1181; Maglio et al., 2002, Protein Engineering 15: 635-642).

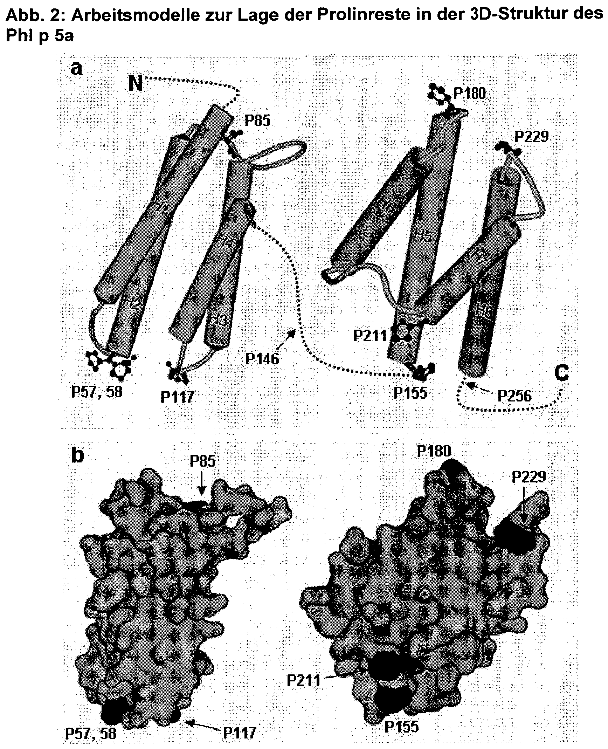

Auf Basis von 3D-Strukturen des Allergens Phl p 6 (RCSB Protein Data Bank Eintrag: 1 NLX) und eines Phl p 5b-Halbmoleküls (RCSB Protein Data Bank Eintrag: 1 L3P) konnte ein Homologiemodell des Phl p 5a generiert werden (Wald et al., 2007, Clin. Exp. Allergy 37:441-450). Nach diesem Modell ist Phl p 5a aus zwei Helixbündeln aufgebaut, wobei aber die genaue Lage der beiden Bündel zueinander durch das Homologiemodell nicht geklärt werden kann (Abb. 2).

Die Spezifische Immuntherapie (SIT) oder Hyposensibilisierung gilt als wirksamer Ansatz zur therapeutischen Behandlung von Allergien (Fiebig 1995 Allergo J. 4 (6):336-339, Bousquet et al., 1998, J. Allergy Clin. Immunol. 102 (4): 558-562); Cox et al., 2007, J. Allergy Clin. Immunol. 120:S25-85; James & Durham, 2008, Clin. Exp. Allergy 38: 1074-1088).

Die klassische Therapieform der Injektionstherapie (SCIT), bei der dem Patienten natürliche Allergenextrakte in steigenden Dosen subkutan injiziert werden, wird seit etwa 100 Jahren erfolgreich angewendet. Dabei wird das Immunsystem des Allergikers wiederholt mit Allergenen konfrontiert, wodurch eine Umprogrammierung des Immunsystems verbunden mit einer Toleranz gegenüber den Allergenen erzielt wird. Nach der Aufnahme der Antigene aus den Allergenpräparaten durch antigenpräsentierende Zellen, werden Peptide der Antigene auf der Zelloberfläche präsentiert. Einige bestimmte Peptide, die sogenannte T-Zell-Epitope enthalten, werden von antigenspezifischen T-Zellen erkannt. Diese Bindung führt unter anderem zur Ausprägung verschiedener Typen von T-Zellen mit regulatorischer Funktion. Im Laufe der SIT führt die regulatorische T-Zell-Antwort zu einer Toleranz gegenüber dem Allergen, der Abregulierung von T_{H}2-Zytokinen, der Wiederherstellung des T_{H}1/T_{H}2-Gleichgewichtes, der Suppression von Allergen-spezifischem IgE, der Induktion von IgG4-, IgG1 und IgA-Antikörpern, der Suppression von Effektorzellen (Mastzellen, Basophile und Eosinophile) sowie der Erneuerung des entzündeten Gewebes (Akdis et al., 2007, J. Allergy Clin. Immunol. 119 (4):780-789; Larchè et al., 2008, Nature Reviews 6:761-771). Die T-Zell-Epitope sind somit für die therapeutische Wirkung der Allergenpräparate bei der Hyposensibilisierung von entscheidender Bedeutung.

Aufgrund der sowohl auf IgE- als auch und T-Zell-Ebene vorhandenen Kreuzreaktivität der Majorallergene der Süßgräser ist eine erfolgreiche Therapie mit einem Allergenextrakt einer einzigen repräsentativen Gräser-Spezies meist ausreichend (Malling et al., 1993, EAACI Position Paper: Immunotherapy, Allergy 48: 9-35; Cox et al., 2007, J Allergy Clin Immunol 120: 25-85).

Neben der subkutanen Immuntherapie kommt eine sublinguale Therapieform, bei der die Allergene oder Allergenderivate über die Mundschleimhaut aufgenommen werden, als Alternative für die Injektionstherapie zur klinischen Erprobung und Anwendung (James & Durham, 2008, Clin. Exp. Allergy 38: 1074-1088).

Eine weitere Möglichkeit ist die Behandlung mit expressionsfähiger DNA, die für die relevanten Allergene kodiert (immuntherapeutische Vakzinierung). Experimentelle Belege für die allergenspezifische Beeinflussung der Immunantwort wurden an Nagern durch Injektion von allergenkodierender DNA erbracht (Hsu et al. 1996, Nature Medicine 2 (5):540-544, Weiss et al., 2006, Int. Arch. Allergy Immunol. 139: 332-345).

Bei all diesen Therapieformen besteht eine grundsätzliche Gefahr von allergischen Reaktionen oder sogar eines anaphylaktischen Schocks (Kleine-Tebbe, 2006, Allergologie, 4:135-156). Um diese Risiken zu minimieren, werden innovative Präparate in Form von Allergoiden eingesetzt. Dabei handelt es sich um chemisch modifizierte Allergenextrakte, die deutlich reduzierte IgE-Reaktivität, jedoch identische T-Zell-Reaktivität im Vergleich zum nicht behandelten Extrakt aufweisen (Fiebig 1995 Allergo J. 4 (6):336-339, Kahlert et al., 1999, Int. Arch. Allergy Immunol, 120: 146-157).

Eine Therapieoptimierung ist mit rekombinant hergestellten Allergenen möglich. Definierte, gegebenenfalls auf die individuellen Sensibilisierungsmuster der Patienten abgestimmte Cocktails von hochreinen, rekombinant hergestellten Allergenen könnten Extrakte aus natürlichen Allergenquellen ablösen, da diese außer den verschiedenen Allergenen eine größere Zahl von immunogenen, aber nicht allergenen Begleitproteinen enthalten. Erste klinische Studien mit rekombinanten Allergenen wurden bereits mit Erfolg durchgeführt (Jutel et al., 2005, J. Allergy Clin. Immunol., 116: 608-613; Valenta & Niederberger, 2007, J. Allergy Clin. Immunol. 119: 826-830).

Realistische Perspektiven, die zu einer sicheren Hyposensibilisierung mit rekombinanten Expressionsprodukten führen können, bieten gezielt mutierte rekombinante Allergene, bei denen IgE-Epitope verändert werden, ohne die für die Therapie essentiellen T-Zell-Epitope zu beeinträchtigen (Schramm et al. 1999, J. Immunol. 162:2406-2414). Diese hypoallergenen Proteine könnten in höheren Dosen während der SIT eingesetzt werden, ohne dabei die Wahrscheinlichkeit von unerwünschten IgE-vermittelten Nebenwirkungen zu erhöhen.

In der Vergangenheit wurden für viele Aeroallergene (u.a. Pollen- und Hausstaubmilben-Allergene) und Nahrungsmittelallergene solche "hypoallergenen" Varianten mit erniedrigter IgE-Bindung publiziert. Ausgehend von den DNA unveränderter Allergene konnte unter anderem durch Fragmentierung, Oligomerisierung, Deletionen, Punktmutationen oder der Neukombination einzelner Abschnitte eines Allergens (DNA-Shuffling) eine rekombinante DNA hergestellt und exprimiert werden (Ferreira et al., 2006, Inflamm. & Allergy - Drug Targets 5: 5-14; Bhalla & Singh, 2008, Trends in Biotechnology 26:153-161).

Bezüglich der Gräserpollen-Allergene wurden hypoallergene Varianten für die Gruppen 1, 2, 5a, 5b, 6, 7 und 12 beschrieben (Ferreira et al., 2006, Inflamm. & Allergy - Drug Targets 5: 5-14; Westritschnig et al., 2007, J. Immunol. 179: 7624-7634).

In mehreren Publikationen sind bisher Ansätze zur Entwicklung hypoallergener Gruppe 5-Allergene beschrieben. Für Phl p 5a und Phl p 5b konnte gezeigt werden, dass die kombinierte Deletion zweier Sequenzabschnitte eine starke Verminderung der IgE-Bindung sowie eine reduzierte Stimulierbarkeit von basophilen Effektorzellen zur Folge hat. Die T-Zell-Reaktivität der Deletionsmutanten war jedoch geringfügig gegenüber der des unveränderten Allergens verändert (Schramm et al., 1999, J. Immunol. 162: 2406-2414; Wald et al., 2007, Clin. Exp. Allergy 37:441-450).

In einer anderen Arbeit wurde das Gruppe 5-Allergen aus *Lolium perenne* (Lol p 5) durch Aminosäuresubstitutionen und/oder kurze Deletionen am C-Terminus verändert (Swoboda et al., 2002, Eur. J. Immunol. 32: 270-280). Die Mutationen beschränkten sich nicht auf einen bestimmten Aminosäuretyp. Die substituierten Aminosäuren waren Lysin, Phenylalanin, Threonin, Valin oder Alanin. Der konzeptionelle Ansatz, durch gezielte Mutation von mehreren Resten einer einzelnen Aminosäure hypoallergene Mutanten zu generieren, ist ebenfalls beschrieben worden. Gelhar et al. beschrieben die Generierung eines rekombinanten Phl p 5b-Fragmentes durch Substitution von zehn an der Proteinoberfläche lokalisierten Lysinresten mit Alanin (Gelhar et al., 2006, Int. Arch. Allergy Immunol. 140:285-294). Eine auf Punktmutationen in Prolinresten basierende Mutationsstrategie, ist jedoch bisher für Gräserpollen-Allergene der Gruppe 5 noch nicht publiziert worden.

Die der vorliegenden Erfindung zugrunde liegende Aufgabe bestand nun in der Bereitstellung neuartiger rekombinanter Varianten der Gruppe 5-Allergene der *Poaceae* auf Protein- und DNA-Ebene, die sich bei weitgehendem Erhalt der T-Zell-Reaktivität durch eine reduzierte IgE-Reaktivität auszeichnen und daher für die kurative und präventive spezifische Immuntherapie sowie die immuntherapeutische DNA-Vakzinierung geeignet sind.

### Beschreibung der Erfindung

Überraschend wurde gefunden, dass Varianten von Gruppe 5-Allergenen der Familie der Süßgräser *(Poaceae),* bei denen die Proline, die in einem Alignment den Prolinen der Positionen 57, 58, 117, 146, 155, 211, 229 in der Aminosäuresequenz des Wildtyp-Phl p 5.0109 entsprechen, einzeln oder in Kombinationen mutiert sind, eine gegenüber den Wildtyp-Allergenen verringerte IgE-Reaktivität und gleichzeitig eine weitgehend erhaltene Reaktivität mit T-Lymphozyten aufweisen und damit hypoallergen sind.

Gegenstand der Erfindung sind demgemäß hypoallergene Varianten von Gruppe 5-Allergenen der Familie der Süßgräser *(Poaceae),* bei denen die Proline, die in einem Alignment den Prolinen der Positionen 57, 58, 117, 146, 155, 211, 229 in der Aminosäuresequenz des Wildtyp-Phl p 5.0109 entsprechen, einzeln oder in Kombinationen mutiert sind.

Besonders bevorzugt sind erfindungsgemäße Allergenvarianten, dadurch gekennzeichnet, dass die Proline deletiert oder substituiert sind.

Bevorzugt sind erfindungsgemäße, hypoallergene Varianten von Gruppe 5-Allergenen aus der Unterfamilie der *Pooideae,* bevorzugt aus den Gruppen *Poodae* und *Triticodae,* bevorzugt vertreten durch *Phleum pratense, Holcus Ianatus*, *Phalaris aquatica, Anthoxanthum odoratum, Dactylis glomerata, Lolium perenne, Poa pratensis, Festuca pratensis, Hordeum vulgare, Secale cereale* und *Triticum aestivum.* Bevorzugt handelt es sich um erfindungsgemäße, hypoallergene Varianten von Tri a 5, Sec c 5 und Hor v 5 aus *Triticum aestivum*, *Secale cereale und Hordeum vulgare.* Besonders bevorzugt sind erfindungsgemäße, hypoallergene Varianten von Gruppe 5-Allergenen der *Poodae.* Vorzugsweise sind diese Gruppe 5-Allergene Poa p 5, Dac g 5, Hol p 5, Lol p 5 und Pha a 5 aus *Poa pratensis, Dactylis glomerata, Holcus Ianatus*, *Lolium perenne* und *Phalaris aquatica* und ganz besonders bevorzugt ist Phl p 5 aus *Phleum pratense.* Erfindungsgemäß sind auch alle natürlich vorkommenden Isomere, Polymorphe und Varianten der vorgenannten Allergene, sowie deren Vorläufer-Proteine.

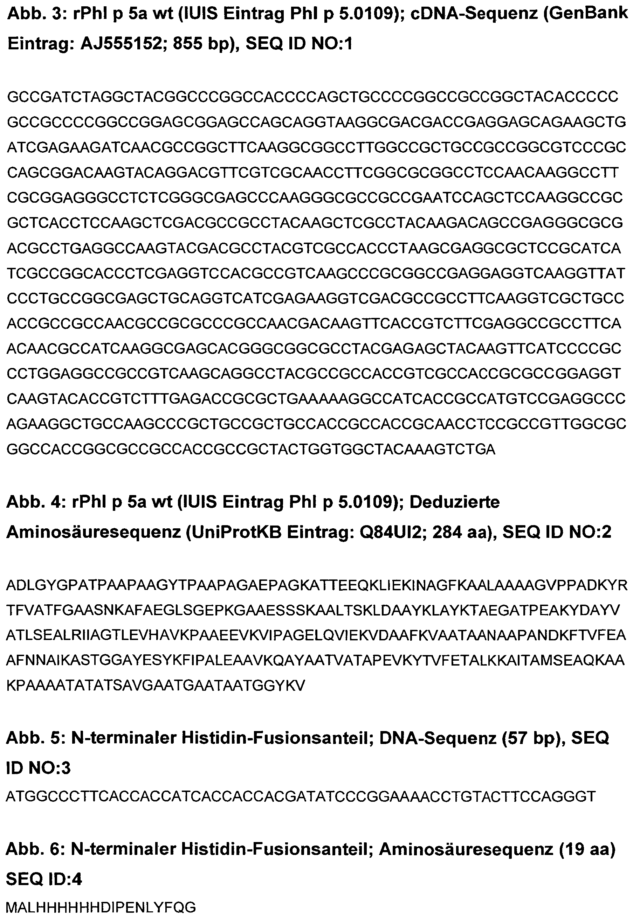

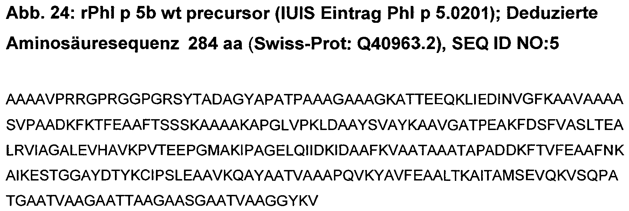

Bevorzugt sind bei den erfindungsgemäßen, hypoallergenen Varianten die Proline mutiert, die in einem Alignment (Abb 1a) den Prolinen der Positionen 57, 58, 117, 146, 155, 211 bzw. 229 in der Aminosäuresequenz des reifen Phl p 5.0109 (Phl p 5 a, UniProtKB Eintrag: Q84UI2) Abb. 3 und 4, SEQ ID NO:1, SEQ ID NO:2) oder dessen Varianten (Abb. 1b) oder des reifen Phl p 5.0201 (Phl p5 b; Swiss-Prot: Q40963.2; Abb. 24, SEQ ID NO: 5) oder dessen Varianten (Abb. 1b), besonders bevorzugt des reifen Phl p 5.0109, entsprechen.

Obwohl bekannt war, dass Proline einen Einfluss auf die Proteinstruktur ausüben können, wurden gezielte Punktmutationen von Prolinresten als Ansatzpunkt für die Generation hypoallergener Mutanten von Allergenen lediglich bei dem Gruppe 2 Hauptallergen der Hausstaubmilbe *Dermatophaogides farinae* (Der f 2, Austausch von Prolinresten durch Alanin) untersucht (Takai et al., 2000, Eur. J. Biochem. 267: 6650-6656). Die IgE-Bindungsfähigkeit und Fähigkeit zur Stimulierung von basophilen Zellen war jedoch bei drei Punktmutanten nur leicht reduziert, während die übrigen drei sich wie das unveränderte Allergen verhielten. Die Prolinmutationen zeigten somit bei Der f 2 keine oder eine nur sehr schwache Reduktion der Allergenität. Weitere Strategien zur Herstellung hypoallergener Mutanten durch Prolin-Austausch-Mutationen sind nicht publiziert. Somit war für den Fachmann nicht zu erwarten, dass Prolinmutationen als Ansatzpunkt für die Generation hypoallergener Mutanten von Allergenen erfolgreich sein würden.

Überdies wurde bisher für kein Allergen untersucht, wie sich eine spezifische Deletion von Prolinresten auf die Gesamt-IgE-Bindungsfähigkeit des Expressionsprodukts auswirkt und welche Auswirkungen auf die Aktivierung von Allergie-relevanten Effektorzellen erfolgen.

In der Aminosäuresequenz von Phl p 5.0109 (UniProtKB: Q84UI2) finden sich 16 Prolinreste (Abb. 1). Die sechs Prolinreste P7, P10, P13, P19, P22 und P27 sind in dem innerhalb der Gruppe 5-Allergene gering konservierten N-terminalen Bereich lokalisiert. Die Proline der Aminosäurepositionen 57, 58, 85, 117, 146, 155, 211, 229 und 256 liegen am Beginn oder am Ende von α-Helices oder in Schleifen, die die α-Helices verbinden und sind bis auf Aminosäure P85 stark konserviert (Abb. 1, 2).

Ausgehend von der Aminosäuresequenz der Phl p 5a-Isoform Phl p 5.0109 werden das rekombinante nicht modifizierte Wildtyp-Allergen (rPhl p 5a wt; Abb. 4, SEQ ID NO:2) sowie gentechnisch veränderte Varianten hergestellt. Analog zum nachfolgend dargestellten Herstellungsverfahren lassen sich auch die Wildtyp-Proteine und die erfindungsgemäßen, hypoallergenen Varianten der weiteren erfindungsgemäßen Gruppe 5-Allergene der Süßgräser, beispielsweise Lol p 5; Poa p 5, Pha a 5, Dac g 5; Hol I 5, Tri a 5 und Hor v 5 herstellen. Dazu werden die Proline, die in einem Alignment den Prolinen der Positionen der Positionen 57, 58, 117, 146, 155, 211, 229 in der Aminosäuresequenz des Wildtyp-Phl p 5 entsprechen, einzeln oder in Kombinationen mutiert, bevorzugt durch Substitution oder Deletion.

Naturgemäß sind über die beschriebenen Variationen von Gruppe 5-Allergenen hinaus auch weitere Veränderungen an anderen Positionen - etwa zur Erhöhung der Hypoallergenität - möglich. Bei diesen Modifikationen kann es sich beispielsweise um Aminosäure-Insertionen, -Deletionen, -Austausche und Aufspaltungen des Proteins in Fragmente sowie Fusionen des Proteins oder seiner Fragmente mit anderen Proteinen oder Peptiden handeln, sowie Multimere durch Fusionen gleicher Proteine oder Fragmente.

Erfindungsgemäße Fragmente umfassen vorzugsweise 20-109 Aminosäuren, bevorzugt 30-100 Aminosäuren, besonders bevorzugt 40-90 Aminosäuren. Erfindungemäße Varianten umfassen überdies Vorläuferproteine wie beispielsweise ProPhl p 5 mit einer vorgeschalteten, natürlichen oder künstlichen Signalsequenz. Erfindungsgemäß sind außerdem Fusionsproteine mit N- oder C-terminalen Fusions-Tags (z.B. His-Tag wie in Abb. 5 und 6, MBP-Tag, Expressionskontrollsequenzen etc.), Hybridmoleküle wie beispielsweise Fusionen mit anderen Allergenen oder ihren hypoallergenen Varianten oder Fusionen von Fragmenten in beliebiger Reihenfolge. Überdies umfassen die erfindungsgemäßen Varianten auch homologe Sequenzen (Polymorphien (SNPs), Isoformen) mit einer Identität der Aminosäuresequenz von wenigstens 80% zum betreffenden Gruppe 5-Wildtyp-Allergen, bevorzugt von wenigstens 90% zum betreffenden Gruppe 5-Wildtyp-Allergen, besonders bevorzugt von wenigstens 95% zum betreffenden Gruppe 5-Wildtyp-Allergen. Bevorzugt werden bei diesen Varianten eine oder wenige Aminosäuren konservativ ausgetauscht, beispielweise eine polare Aminosäure durch eine andere polare Aminosäure oder eine neutrale durch eine andere neutrale subtituiert, jedoch sind auch Varianten durch nicht-konservativen Austausch erfindungsgemäß. Multimere umfassen bevorzugt Dimere oder Trimere der erfindungsgemäßen hypoallergenen Varianten verbunden durch eine Linkersequenz oder direkt fusioniert.

Beispiele für Isoformen sind die Allergene Phl p 5a und Phl p 5b, bei denen einzelne für die erfindungsgemäße Wirkung nicht relevante Aminosäuren ausgetauscht sind, bzw. bei denen Bereiche in der Aminosäresequenz fehlen oder hinzugefügt sind (siehe Abb 1a). Diese Wildtyp-Allergen Isoformen weisen beispielsweise eine Identität der Aminosäuresequenz von 63% - 71% auf. Weitere Beispiele für erfindungsgemäße Varianten sind Varianten der Wildtyp-Allergen Isoformen Phl p 5a und Phl p 5b, wie beispielweise Phl p 5.0109, Phl p 5.0201, Phl p 5.0204, Phl p 5.0206, Phl p 5.0207 und dergleichen und weitere Varianten mit Austauschen einer oder mehrer Aminosäuren, Fehlen einer oder mehrer Aminosäuren am N- und/oder C-Terminus oder mit entsprechenden Deletionslücken innerhalb der Aminosäurensequenz. Ebenso erfindungsgemäß sind Varianten mit Insertionen einzelner oder mehrer Aminosäuren vereinzelt in verschiedenen Positionen oder als Gruppe in einer Position innerhalb der Aminosäuresequenz oder am N- und/oder C-Terminus.

Erfindungsgemäßer Gegenstand sind somit auch hypoallergene Varianten von Gruppe 5-Allergenen der Süßgräser (Poaceae), dadurch gekennzeichnet, dass es sich um ein Fragment oder eine Variante einer erfindungsgemäßen, hypoallergenen Variante oder um ein Multimer einer oder mehrerer erfindungsgemäßer, hypoallergener Varianten handelt oder dadurch gekennzeichnet, dass eine oder mehrere erfindungsgemäße, hypoallergene Varianten oder deren Fragmente, Varianten oder Multimere Bestandteil eines rekombinanten Fusionsproteins sind.

Gegenstand der Erfindung ist außerdem ein DNA-Molekül, das für eine erfindungsgemäße, hypoallergene Variante kodiert.

Ein weiterer Gegenstand der Erfindung ist ein rekombinanter Expressionsvektor, enthaltend ein solches erfindungsgemäßes DNA-Molekül funktionell verbunden mit einer Expressions-Kontrollsequenz. Unter einer Expressions-Kontrollsequenz versteht man beispielsweise einen Promotor oder einen Sequenzabschnitt mithilfe dessen die Expression des Zielproteins beeinflusst wird und der in funktioneller Verbindung zum Zielgen steht, jedoch nicht zwangsläufig dem Zielgen in direkter Nachbarschaft lokalisiert sein muss.

Ein erfindungsgemäßer Gegenstand ist auch ein nicht-menschlicher Wirtsorganismus, transformiert mit einem erfindungsgemäßen DNA-Molekül oder einem erfindungsgemäßen Expressionsvektor.

Ein erfindungsgemäßer Gegenstand ist ein Verfahren zur Herstellung einer erfindungsgemäßen, hypoallergenen Variante durch Kultivierung eines erfindungsgemäßen nicht-menschlichen Wirtsorganismus und Gewinnung der entsprechenden Allergenvariante aus der Kultur.

Geeignete nicht-menschliche Wirtsorganismen können pro- oder eukaryontische, ein- oder mehrzellige Organismen wie Bakterien oder Hefen sein. Ein erfindungsgemäß bevorzugter Wirtsorganismus ist E. *coli*.

Der Einfluss der Deletion einzelner oder zweier eng benachbarter Proline auf die IgE-Bindungsfähigkeit des Phl p 5a kann durch Deletion der Proline 57 + 58, von Prolin 85, Prolin 117, Prolin 146 + 155, Prolin 180, Prolin 221, Prolin 229 und von Prolin 256 untersucht werden. Diese Proline sind im Phl p 5-Wildtypprotein in den Schleifenregionen am Beginn oder am Ende von α-Helices lokalisiert (Abb. 3). Analog dazu kann der Einfluss von Prolinmutationen in den entsprechenden homologen Positionen der weiteren erfindungsgemäßen Gruppe 5-Allergene der Süßgräser, beispielsweise Poa p 5 und Lol p 5 auf die IgE-Bindungsfähigkeit hin untersucht werden.

Zu schnelleren ausbeutereichen Aufreinigung wird die kodierende DNA für diese Untersuchungen mit einer Sequenz kodierend für einen N-terminalen Hexa-Histidin-Fusionsanteil (+ 6His) versehen (Abb. 5, SEQ ID NO:3; Abb. 6, SEQ ID NO:4). Die Tag-freien, für pharmazeutische Zwecke einsetzbaren, erfindungsgemäßen Varianten und Wildtypproteine werden ebenfalls nach Standardmethoden gereinigt und bestätigen die Ergebnisse der His-tag Proteine.

Dementsprechend werden Sequenzen kodierend für die Proteine rPhl p 5a d[P57, P58] + 6His , rPhl p 5a d[P85] + 6His, rPhl p 5a d[P117] + 6His, rPhl p 5a d[P146, P155] + 6His, rPhl p 5a d[P180] + 6His, rPhl p 5a d[P211] + 6His, rPhl p 5a d[P229] + 6His und rPhl p 5a d[P256] + 6His hergestellt. Die Sequenzen können in allen bekannten eukaryontischen und prokaryontischen Expressionssystemen, vorzugweise in *E*. *coli* exprimiert werden. Im Anschluss werden die Proteine als lösliche Monomere über Standardmethoden gereinigt. Schließlich kann Reinheit durch Analyse in einem denaturierenden Polyacrylamid-Gel (SDS-PAGE) überprüft werden.

Die analytische Gelfiltration (SEC) mit Kopplung eines Refraktometers (RI-Detektor) und eines Mehrwinkellichtstreu-Detektors (MALS-Detektor) erlaubt die Online-Bestimmung der Molekülmasse der eluierten Proteine (SEC/MALS/RI-Methode).

Die erfindungsgemäßen, rekombinanten Varianten können überdies hinsichtlich ihrer Bindungsfähigkeit an humane IgE-Antikörper durch IgE-Inhibitionstests (EAST) untersucht werden. Bei diesem Verfahren kann die Allergen/IgE-Interaktion in Lösung untersucht werden, womit störende Maskierungen von Epitopen der Testsubstanz, etwa durch Immobilisierung an eine Membran, ausgeschlossen werden können.

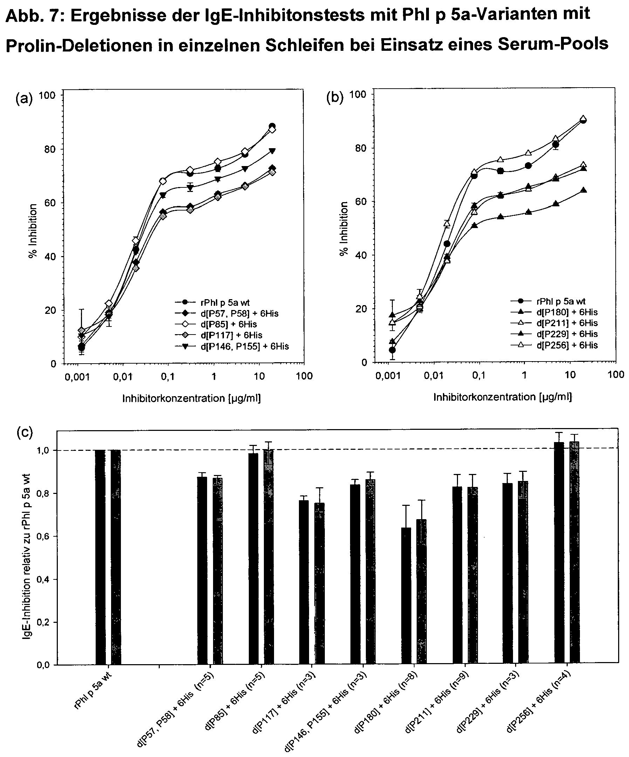

Es kann eine verringerte IgE-Bindung der Mutanten rPhl p 5a d[P57, P58] + 6His, rPhl p 5a d[P117] + 6His, rPhl p 5a d[P146, P155] + 6His, rPhl p 5a d[P180] + 6His, rPhl p 5a d[P211] + 6His und rPhl p 5a d[P229] + 6His im Vergleich zu dem nicht modifizierten rPhl p 5a wt festgestellt werden (Abb. 7).

Dies beweist grundsätzlich, dass die Deletion von Prolinresten der Gruppe 5-Allergene die IgE-Bindungsfähigkeit dieser Allergene reduziert. Andererseits hat nur die Deletion bestimmter Proline eine erniedrigte IgE-Bindung zu Folge.

Ein weiterer Gegenstand der vorliegenden Erfindung sind daher hypoallergene Varianten von Gruppe 5-Allergenen der Familie der Süßgräser *(Poaceae),* bei denen die Proline, die in einem Alignment den Prolinen der Positionen 57, 58, 117, 146, 155, 211 oder 229 in der Aminosäuresequenz des Wildtyp-Phl p 5.0109 entsprechen, einzeln mutiert sind.

Ein bevorzugter Gegenstand der vorliegenden Erfindung sind hypoallergene Varianten von Phl p 5, Poa p 5, Lol p 5, Dac g 5, Hol I 5, Pha a 5, bei denen die Proline, die in einem Alignment den Prolinen der Positionen 57, 58, 117, 146, 155, 211 oder 229 in der Aminosäuresequenz des Wildtyp-Phl p 5.0109 entsprechen, einzeln mutiert sind.

Ein besonders bevorzugter Gegenstand der vorliegenden Erfindung sind hypoallergene Varianten von Phl p 5, bei denen die Proline, die in einem Alignment den Prolinen der Positionen 57, 58, 117, 146, 155, 211 oder 229 in der Aminosäuresequenz des Wildtyp-Phl p 5.0109 entsprechen, einzeln mutiert sind.

Ein weiterer bevorzugter Gegenstand der vorliegenden Erfindung sind erfindungsgemäße, hypoallergene Varianten von Gruppe 5-Allergenen der Familie der Süßgräser *(Poaceae),* bei denen die Proline, die in einem Alignment den Prolinen der Positionen 57, 58, 117, 146, 155, 211 oder 229 in der Aminosäuresequenz des Wildtyp-Phl p 5.0109 entsprechen, einzeln entfernt sind.

Ein weiterer Gegenstand der vorliegenden Erfindung sind außerdem erfindungsgemäße, hypoallergene Varianten von Gruppe 5-Allergenen der Familie der Süßgräser *(Poaceae),* bei denen die Proline, die in einem Alignment den Prolinen der Positionen 57, 58, 117, 146, 155, 211 oder 229 in der Aminosäuresequenz des Wildtyp-Phl p 5.0109 entsprechen, einzeln substituiert sind. Hier wird Prolin beispielhaft durch Leucin (L) ausgetauscht. Erfindungsgemäß können jedoch die erfindungsgemäßen Proline durch jede Aminosäure ausgetauscht sein.

Erfindungsgemäß sind insbesondere die hypoallergenen Varianten rPhl p 5a d[P57], rPhl p 5a d[P58], rPhl p 5a d[P57, P58], rPhl p 5a d[P117], rPhl p 5a d[P146], rPhl p 5a d[P155], rPhl p 5a d[P146, P155], rPhl p 5a d[P180], rPhl p 5a d[P211], rPhl p 5a d[P229], rPhl p 5a P57L, rPhl p 5a P58L, rPhl p 5a P57, P58L, rPhl p 5a P117L, rPhl p 5a P146L, rPhl p 5a P155L, rPhl p 5a P146L, P155L , rPhl p 5a P180L, rPhl p 5a P211L und rPhl p 5a P229L, und dergleichen einschließlich aller nachfolgend beschriebenen, erfindungsgemäßen, hypoallergenen Varianten, wobei die Nummerierung der Sequenz des Phl p 5.0109 folgt.

Weiterhin sind diese Beispiele nicht auf Varianten des Phl p 5a beschränkt sondern betreffen auch insbesondere Phl p 5b, Poa p 5, Hol I 5, Pha a 5, Ant o 5, Dac g 5, Lol p 5, Fes p 5, Hor v 5, Sec c 5, Tri a 5 und die Gruppe 5-Allergene aller übrigen Süßgräser. Besonders hervorzuheben sind entsprechende Varianten von Poa p 5, Lol p 5, Dac g 5, Hol I 5, Pha a 5 und Phl p 5b.

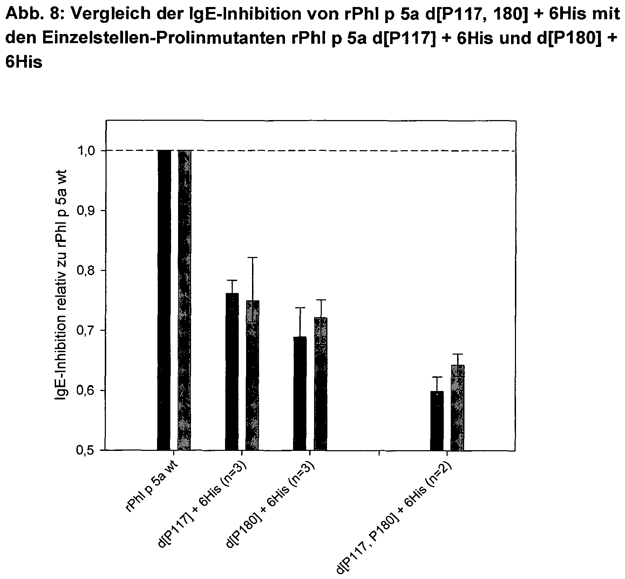

Überraschenderweise zeigen Varianten mit Kombinationen von Deletionen aus der Gruppe der Mutationen d[P57, P58], d[P117], d[P146, P155], d[P180], d[P211] und d[P229] eine deutlich stärker verringerte IgE-Bindungsfähigkeit im EAST-Verfahren, wie am Beispiel der Variante rPhl p 5a d[P117, 180] + 6His gezeigt wird (Abb. 8).

Ein weiterer Gegenstand der vorliegenden Erfindung sind daher hypoallergene Varianten von Gruppe 5-Allergenen der Familie der Süßgräser (Poaceae), bei denen die Proline, die in einem Alignment den Prolinen der Positionen 57, 58, 117, 146, 155, 180, 211, 229 in der Aminosäuresequenz des Wildtyp-Phl p 5.0109 entsprechen, in Kombinationen mutiert sind. Bevorzugt sind Mutationen durch Deletion und durch Substitution durch andere Aminosäuren. Dabei kann jede Aminosäure zum Austausch gegen Prolin gewählt werden.

Ein bevorzugter Gegenstand der vorliegenden Erfindung sind hypoallergene Varianten von Phl p 5, Poa p 5, Lol p 5, Dac g 5, Hol I 5, Pha a 5, bei denen die Proline, die in einem Alignment den Prolinen der Positionen 57, 58, 117, 146, 155, 180, 211, 229 in der Aminosäuresequenz des Wildtyp-Phl p 5.0109 entsprechen, in Kombinationen mutiert sind.

Ein besonders bevorzugter Gegenstand der vorliegenden Erfindung sind hypoallergene Varianten von Phl p 5, bei denen die Proline, die in einem Alignment den Prolinen der Positionen 57, 58, 117, 146, 155, 180, 211, 229 in der Aminosäuresequenz des Wildtyp-Phl p 5.0109 entsprechen, in Kombinationen mutiert sind.

Weiterhin sind diese Beispiele nicht auf Varianten des Phl p 5 beschränkt sondern betreffen auch insbesondere Poa p 5, Hol I 5, Pha a 5, Ant o 5, Dac g 5, Lol p 5, Fes p 5, Hor v 5, Sec c 5, Tri a 5 und die Gruppe 5-Allergene aller übrigen Süßgräser. Besonders hervorzuheben sind entsprechende Varianten von Phl p 5a, Phl p 5b, Poa p 5, Lol p 5, Dac g 5, Hol I 5, Pha a 5.

Besonders bevorzugt sind erfindungsgemäße, hypoallergene Varianten bei denen die Proline 57, 58, 117, 146, 155, 180, 211, 229 in Kombinationen entfernt sind. Besonders bevorzugt sind auch erfindungsgemäße, hypoallergene Varianten bei denen die Proline 57, 58, 117,146, 155, 180, 211, 229 in Kombinationen substituiert sind.

Besonders bevorzugt sind außerdem erfindungsgemäße, hypoallergene Varianten bei denen die Proline 57, 58, 117, 146, 155, 180, 211, 229 in Kombinationen entfernt und/oder substituiert sind. Erfindungsgemäß sind daher bespielsweise die hypoallergenen Varianten rPhl p 5a d[57, 58, 117], rPhl p 5a d[57, 58, 146], rPhl p 5a d[57, 58, 150], rPhl p 5a d[57, 58, 180], rPhl p 5a d[57, 58, 211], rPhl p 5a d[57, 58, 229], rPhl p 5a d[117, 146, 155], rPhl p 5a d[117, 146, 155, 180], rPhl p 5a d[117, 146, 155, 229], rPhl pa 5 P117L, P146L, P155L, P211L, rPhl p 5a d[117, 180, 229] P211L, rPhl p 5a d[117, 180, 229] P211L, rPhl p 5a d[57, 58, 117, 180, 229] P211L und dergleichen einschließlich aller nachfolgend beschriebenen, erfindungsgemäßen, hypoallergenen Varianten, wobei die Nummerierung der Sequenz des Phl p 5.0109 folgt.

Hier wird Prolin beispielhaft durch Leucin (L) ausgetauscht. Erfindungsgemäß können jedoch die erfindungsgemäßen Proline durch jede Aminosäure ausgetauscht werden. Erfindungsgemäß sind demnach alle aufgeführten oder denkbaren hypoallergenen Varianten bei denen ein oder mehrere erfindungsgemäße Proline durch eine andere Aminosäure substituiert sind. Weiterhin sind diese Beispiele nicht auf Varianten des Phl p 5 beschränkt sondern betreffen auch insbesondere Phl p 5b, Poa p 5, Hol I 5, Pha a 5, Ant o 5, Dac g 5, Lol p 5, Fes p 5, Hor v 5, Sec c 5, Tri a 5 und die Gruppe 5-Allergene aller übrigen Süßgräser. Besonders bevorzugt sind jedoch alle aufgeführten erfindungsgemäßen, hypoallergenen Varianten von *Phleum pratense* Phl p 5a und Phl p 5b, insbesondere basierend auf Phl p 5.0109.

Auf Basis dieser Erkenntnis können die Varianten rPhl p 5a d[P57, P58, P85, P117, P146, P155, P180, P211, P229, P256] + 6His (Kurzform: MPV.1 + 6His) und rPhl p 5a d[P57, P58, P117, P146, P155, P180, P211, P229] + 6His (Kurzform: MPV.2 + 6His) hergestellt werden, die eine möglichst große Anzahl an kombinierten Deletionen enthalten.

Diese Proteine werden bei ihrer Expression als unlösliche Aggregate (inclusion bodies) in den *E*. *coli-Zellen* abgelegt. Inclusion Bodies werden meist in denaturierenden Agenzien wie 6-8 molarer Guanidiniumhydrochlorid- oder Harnstofflösung solubilisiert und später in eine nicht-denaturierende wässrige Lösung überführt. Diese Überführung in eine nicht-denaturierende Lösungsmittelumgebung stellt einen entscheidenden Schritt bei der Proteinreinigung dar. In der Regel können nur solche Proteine in Endformulierungen von Therapeutika aufgenommen werden, die sich nach diesem Prozess löslich verhalten. Eine industriell verwendete Routinemethode zur Überführung der denaturiert-solubilisierten Proteine in das wässrige Lösungsmittel ist die Methode der "Schnellen Verdünnung" (rapid filution). Dabei werden die in einem denaturierenden Agens gelösten Proteine in ein großes Volumen des nicht-denaturierenden Lösungsmittels gegeben, wobei das denaturierende Agens stark ausverdünnt wird.

Das Löslichkeitsverhalten der hier beschriebenen Mutanten kann systematisch untersucht werden. Dabei werden die in E. coli hergestellten und in Guanidiniumhydrochlorid gelösten Inclusion Bodies in zehn verschiedenen wässrigen gepufferten Lösungen verdünnt und anschliessend der Grad der Löslichkeit durch UV-Vis Spektroskopie bestimmt.

Bei der UV-Vis Spektroskopie wird ein Absorptionsspektrum der Proteinlösung im Wellenlängenbereich von 240-800 nm aufgenommen. Unlösliche Aggregate in Proteinlösungen absorbieren im Bereich >300 nm Wellenlänge, während hochlösliche Proteine in diesem Bereich nicht absorbieren.

Die Testlösungen umfassen einen weiten pH-Bereich (4,5 - 9,0) und beinhalten zum Teil stabilisierende Additive. Die Additive Glyzerin, Tween 80 und L-Arginin-Monohydrochlorid werden sehr oft als Hilfe bei der Solubilisierung schwierig herzustellender, rekombinanter Proteine eingesetzt. Sie repräsentieren die drei wichtigsten Gruppen von solchen Co-Solventien: Glyzerin als Polyalkohol, Tween als Nicht-Ionisches Tensid und L-Arginin-Monohydrochlorid als Aminosäurederivat.

Die Varianten MPV.1 + 6His und MPV.2 + 6His können durch Einsatz des denaturierenden Agens Guanidiniumhydrochlorid zwar solubilisiert werden, aber die systematische Untersuchung zur Löslichkeit zeigt, dass einen anschließende Überführung der Proteine in eine Guanidiniumhydrochlorid freie Formulierung stets mit der Bildung von unlöslichen Proteinaggregaten einhergeht und somit nicht möglich ist (Tab. 1, 2).

Während der zur DNA-Synthese durchgeführten PCR-Experimente entsteht im vorliegenden Beispiel durch mehrere Polymerasefehler eine DNA, kodierend für das Protein rPhl p 5a d[P57, P58, P229] K61 E, E205K, P211 L + 6His (Kurzform: MPV.3 + 6His). Diese DNA wird ebenfalls in E. *coli* exprimiert. Das Protein wird wie die Varianten MPV.1 und MPV.2 als Inclusion Bodies in der *E*. *coli*-Zelle abgelegt.

Das Protein MPV.3 + 6His kann jedoch überraschend leicht in eine nicht-denaturierende Pufferlösung überführt werden (Tab. 3).

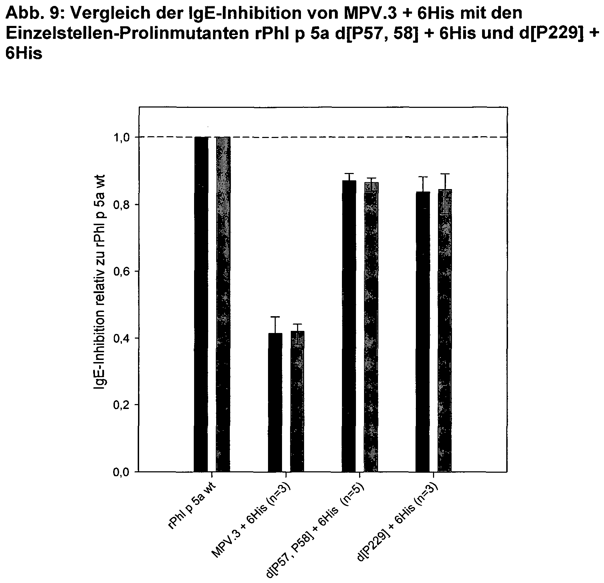

EAST-IgE-Inhibitionstests zeigen eine deutlich verringerte Bindungsfähigkeit des Proteins an IgE-Antikörper im Vergleich zu den Varianten rPhl p 5a d[P57, P58] + 6His und rPhl p 5a d[P229] + 6His, die Prolindeletionen in einer nur Schleife besitzen (Abb. 9).

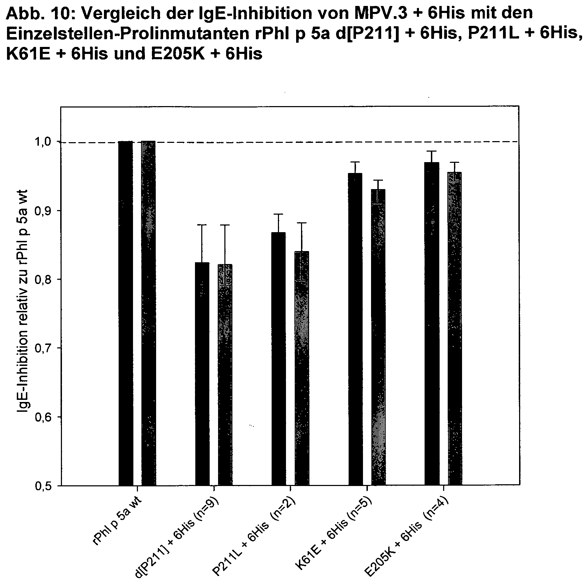

Zur weiteren Charakterisierung der Mutationen K61 E, E205K und P211 L der Variante MPV.3 + 6His werden die DNA der drei Varianten rPhl p 5a K61E + 6His, rPhl p 5a E205K + 6His und rPhl p 5a P211 L + 6His hergestellt und im EAST untersucht. Alle drei Mutanten zeigen eine leicht verringerte IgE-Bindungsfähigkeit, was auf einen synergistischen Einfluss aller Mutationen auf die reduzierte Allergenität von MPV.3 + 6His hinweist (Abb. 10). Dabei zeigt sowohl die Deletion, als auch der Austausch des Prolins 211 einen deutlich stärkeren Einzeleffekt als die Mutationen K61 E oder E205K (Abb. 10).

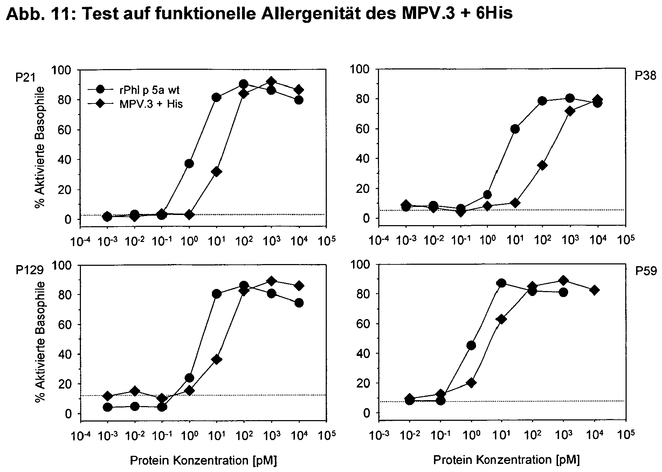

Mittels eines Tests mit basophilen Granulozyten, die aus dem Vollblut von Gräserpollenallergikern gewonnen wurden, wird die Auswirkung der reduzierten IgE-Bindungsfähigkeit von MPV.3 + 6His auf die Aktivierung von humanen Effektorzellen *in vitro* untersucht. Bei gleicher Konzentration des rPhl p 5a wt und des MPV.3 + 6His zeigt letzteres eine geringere Aktivierung von basophilen Granulozyten. Dies weist auf eine funktionell reduzierte Allergenität des MPV.3 + 6His hin (Abb. 11).

Die zufällig generierte DNA der Variante MPV.3 + 6His dient nun als Ausgangspunkt für die gezielte Konstruktion weiterer Varianten. Dabei sollte die hohe Löslichkeit von MPV.3 + 6His erhalten und die IgE-Bindungsfähigkeit durch Einfügen zusätzlicher Prolinmutationen weiter reduziert werden.

Zunächst wird die DNA der Varianten rPhl p 5a d[P57, P58, P117, P146, P155, P180, P229] K61E, E205K, P211L (Kurzform: MPV.4) und rPhl p 5a d[P57, P58, P117, P180, P229] K61E, E205K, P211L (Kurzform: MPV.5) hergestellt. Diese DNA wird sowohl mit als auch ohne Histidin-Fusionsanteil konstruiert und in *E. coli* exprimiert. Die Proteine können durch eine einfache Reinigungsprozedur aus den Inclusion Bodies der *E*. *coli*-Zellen als lösliche Proteine gereinigt werden.

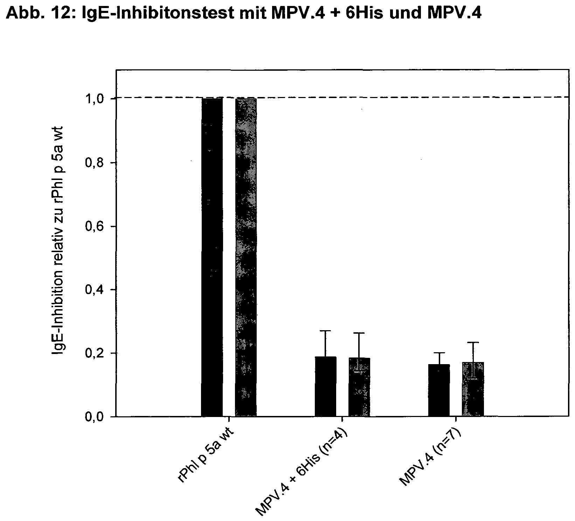

Da sowohl die Proteine mit als auch ohne Fusionsanteil löslich gereinigt werden können, ist die Löslichkeit nicht von dem Vorhandensein des Histidin-Fusionsanteils abhängig. Der Histidin-Fusionsanteil hat ebenso keinen Einfluß auf die IgE-Bindungsfähigkeit wie IgE-Inhibitionstests mit Proteinen mit und ohne Fusionsanteil zeigen (Abb. 12).

Die DNA der Varianten rPhl p 5a d[P57, P58, P117, P146, P155, P180, P229] P211L (Kurzform: MPV.6) und rPhl p 5a d[P57, P58, P117, P180, P229] P211 L (Kurzform: MPV.7) werden ausschließlich ohne Fusionsanteile generiert und in *E*. *coli* exprimiert. Sie können ebenfalls aus Inclusion Bodies durch eine einfache Reinigungsprozedur als hochlösliche Proteine gereinigt werden.

Die Variante MPV.6 unterscheidet sich von der Variante MPV.2 nur durch die Veränderung an Position 211. Statt der Deletion von P211 liegt bei MPV.6 ein Aminosäureaustausch zu Leucin vor. Das extrem unterschiedliche Löslichkeitsverhalten hängt somit direkt von Position 211 ab. Alle Mutanten, die die Mutation P211 L tragen (MPV.3-MPV.7), zeichnen sich durch hohe Löslichkeit in verschiedenen wässrigen Formulierungen aus, während die Mutanten mit einer Deletion an Position 211 unlöslich bleiben (Tab. 1 - Tab. 5).

Gegenstand der vorliegenden Erfindung sind daher erfindungsgemäße, hypoallergene Varianten der Gruppe 5-Allergene der Süßgräser, bei denen der Prolinrest der dem reifen Phl p 5.0109 entsprechenden Aminosäureposition 211 nicht deletiert, sondern durch eine andere Aminosäure ersetzt wird.

Gegenstand der vorliegenden Erfindung sind daher erfindungemäße hypoallergene Varianten der Gruppe 5-Allergene der Süßgräser, bei denen der Prolinrest, der in einem Alignment Prolin 211 in der Aminosäuresequenz des Wildtyp-Phl p 5 bzw. des reifen Phl p 5.0109 entspricht, nicht deletiert sondern durch Leucin ersetzt wird.

In der analytische Gelfiltration (SEC) werden Proteinspezies nicht auschließlich entsprechend ihrem Molekulargewicht (molare Masse) getrennt, hier spielen die Konformation, der spezifischen hydrodynamischen Radius und mögliche Wechselwirkungen mit der Matrix ebenfalls eine wichtige Rolle. Eine echte Online-Bestimmung des Molekulargewichtes kann durch die Kopplung eines Refraktometers (RI-Detektor) und eines Mehrwinkellichtstreu-Detektors (MALS-Detektor) an das SEC-Chromatographiesystem erreicht werden (SEC/MALS/RI-Methode). Dabei wird die zum Meßzeitpunkt gegebene Konzentration eines Partikels durch den RI-Detektor bestimmt und die Lichtstreuung des Partikels durch den MALS-Detektor aufgezeichnet (Wen et al., 1996, Anal. Biochem. 240:155-166). Durch SEC/MALS/RI können Monomere, Dimere, Multimere und Aggregate eindeutig nachgewiesen.

Das Ergebnis der SEC/ MALS/ RI von MPV.5 und MPV.7 zeigt, dass diese Varianten rein und als lösliche Monomere vorliegen (Abb. 13; Abb. 14; Tab. 6). Die beiden Varianten MPV.4 und MPV.6 werden jeweils als ein Gemisch aus löslichen Monomeren und Dimeren nachgewiesen (Abb. 15; Abb. 16; Tab. 6). Die Varianten MPV.5 und MPV.7 unterscheiden sich von den Varianten MPV.4 und MPV.6 jeweils nur an Position 146 und 155. Die Proline P146 und P155 sind bei MPV.5 und MPV.7 vorhanden. Das zeigt, dass die Deletion d[P146, P155] eine starke Neigung zur Dimerisierung der entsprechenden Varianten bewirkt.

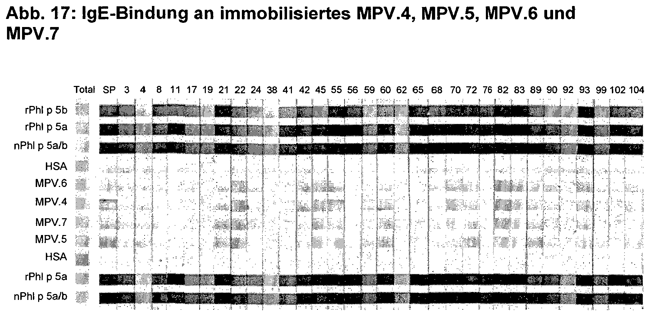

Die IgE-Bindungsfähigkeit der optimierten Varianten MPV.4 bis MPV.7 wird zunächst durch ein Verfahren untersucht, bei dem die Proteine an einer Nitrocellulose-Membran immobilisiert und mit IgE-Antikörper enthaltenden Seren von individuellen Gräserpollenallergikern inkubiert werden. Die Allergenvarianten-Antikörperkomplexe werden anschließend durch eine enzymatische Reaktion angefärbt. In diesem Verfahren zeigt sich eine sehr stark verminderte IgE-Bindungsfähigkeit aller vier Proteine (Abb. 17).

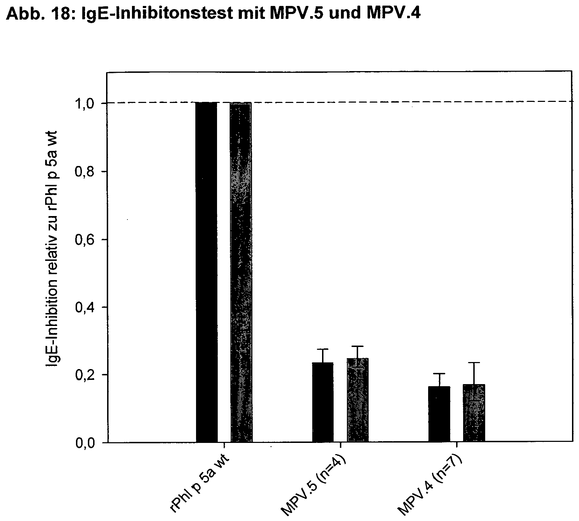

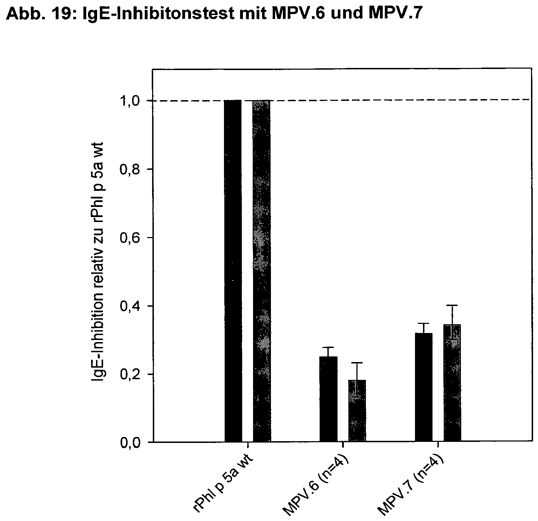

Dieses Ergebnis wird durch einen EAST-IgE-Inhibitionstest mit einem repräsentativen humanen Allergiker-Serumpool bestätigt, wobei die Varianten MPV.4 und MPV.6 eine jeweils etwas geringere IgE-Bindung aufweisen als die korrespondierenden Mutanten MPV.5 und MPV.7 ohne die Mutation d[P146, P155] (Abb. 18; Abb. 19). Die geringere IgE-Bindung bei vorhandener Mutation d[P146, P155] kann wahrscheinlich der Dimerisierungstendenz dieser Varianten zugeschrieben werden, die möglicherweise zu einer Verdeckung von IgE-Epitopen an den Kontaktflächen der Dimerisierungspartner führt.

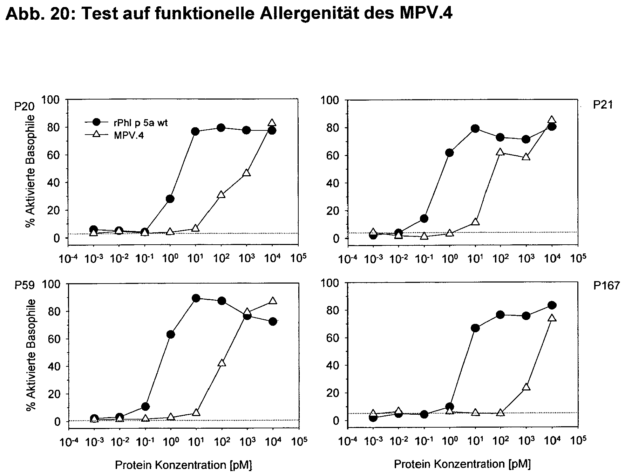

Die Testergebnisse zur Aktivierung von basophilen Granulozyten zeigen für alle vier Varianten eine funktionell stark reduzierte Allergenität (Abb. 20; Abb. 21; Abb. 22; Abb. 23).

Gegenstand der vorliegenden Erfindung sind daher erfindungsgemäße, hypoallergene Varianten der Gruppe 5-Allergene der Süßgräser, dadurch gekennzeichnet, dass das Prolin, das in einem Alignment dem Prolin 211 des Wildtyp-Phl p 5.0109 entspricht, nicht deletiert, sondern durch eine andere Aminosäure ersetzt wird.

Ein bevorzugter Gegenstand der Erfindung sind somit auch erfindungsgemäße, hypoallergene Varianten der Gruppe 5-Allergene der Süßgräser, bei denen die Proline, die in einem Alignment den Prolinen der Positionen 57, 58, 117, 180 und 229 in der Aminosäuresequenz des Wildtyp-Phl p 5.0109 entsprechen, einzeln oder in Kombinationen mutiert, bevorzugt deletiert sind und Prolin 211 durch Leucin ersetzt ist.

Ein bevorzugter Gegenstand der Erfindung sind somit auch erfindungsgemäße, hypoallergene Varianten der Gruppe 5-Allergene der Süßgräser, bei denen die Proline, die in einem Alignment den Prolinen der Positionen 57, 58 und 229 in der Aminosäuresequenz des Wildtyp-Phl p 5.0109 entsprechen, deletiert sind und Prolin 211 durch Leucin ersetzt ist.

Gegenstand der vorliegenden Erfindung sind demnach auch erfindungsgemäße, hypoallergene Varianten bei denen die Proline, die in einem Alignment den Prolinen der Positionen 146 und 155 in der Aminosäuresequenz des Wildtyp-Phl p 5.0109 entsprechen, nicht mutiert werden. Diese liegen bevorzugt als Monomer vor.

Erfindungsgemäß sind auch alle vorgenannten erfindungsgemäßen, hypoallergenen Varianten, bei denen zusätzlich die Aminosäuren, die in einem Alignment Lysin der Position 61 und Glutaminsäure der Position 205 in der Aminosäuresequenz des Wildtyp-Phl p 5 entsprechen, einzeln oder in Kombinationen mutiert sind. Bevorzugt sind diese Aminosäuren substituiert.

Die der Wirksamkeit der spezifischen Immuntherapie zugrunde liegende T-Zell-Reaktivität wird durch einen Proliferationstest mit Phl p 5-spezifischen T-Lymphozyten von Gräserpollenallergikern *in vitro* überprüft. Beispielhaft sind hier die Ergebnisse der Allergenvarianten MPV.4 und MPV.7 beschrieben. Die dimerisierende Variante MPV.4 trägt alle hier untersuchten Mutationen (d[P57, P58, P117, P146, P155, P180, P229] K61E, E205K, P211L) und repräsentiert somit ein Molekül mit maximal veränderter Aminosäuresequenz. Die Mutante MPV.7 wird als Beispiel für eine monomere Form mit reinen Prolin-Mutationen gewählt. Beide Mutanten zeigen eine mit dem unveränderten Allergen vergleichbar gute T-Zell-Reaktivität (Tab. 7; Tab. 8). Der Erhalt der entscheidenden T-Zell-Epitope ermöglicht eine immuntherapeutische Verwendung der beschriebenen Varianten.

T-Helfer-Lymphozyten reagieren mit Peptidfragmenten der Allergene, die durch Abbauprozesse in Antigenpräsentierenden Zellen (APZ) entstehen und gebunden an MHC-Klasse II-Moleküle an der Oberfläche der APZ präsentiert werden. Die Peptide haben in der Regel eine Länge von 13-18 Aminosäuren, können aufgrund der seitlich offenen Bindestelle des MHC-Klasse 2 jedoch auch länger sein. Die Hauptkontaktpunkte des Peptides mit dem MHC-Klasse Molekül sind in einer Kernsequenz von etwa 7-10 Aminosäuren zu finden. Die allergenspezifische Aktivierung der T-Helfer-Lymphozyten ist die Vorrausetzung für deren Proliferation und funktionellen Differenzierung (z.B. Treg, T_{H}1 und T_{H}2). Die Fähigkeit eines Allergens oder einer Allergenvariante zur Stimulierung von allergenspezifischen T-Lymphozyten wird als ein Schlüssel für deren therapeutische Wirksamkeit angesehen.

Alle auf Basis der von Phl p 5 beziehungsweise von Phl p 5.0109 erzeugten und hier dargestellten Allergenvarianten zeigen in Experimenten einen weitgehenden Erhalt entscheidender T-Zell-Epitope.

Es werden somit erstmals Varianten der Gruppe-5-Allergene der Poaceae beschrieben, die durch die Veränderung von Prolinresten neuartige Proteineigenschaften aufweisen. Die betroffenen Prolinreste sind in Schleifenregionen lokalisiert. Nur die Veränderung bestimmter Prolinreste führt zu den neuartigen Varianten, die sich bei weitgehendem Erhalt der T-Zell-Reaktivität durch eine reduzierte IgE-Reaktivität auszeichnen und daher für die kurative und präventive spezifische Immuntherapie geeignet sind. Entsprechende DNA-Moleküle sind für die immuntherapeutische Vakzinierung geeignet sind.

Gegenstand der vorliegenden Erfindung sind deshalb die beschriebenen, erfindungsgemäßen Allergenvarianten, DNA-Moleküle und rekombinanten Expressionsvektoren als Arzneimittel.

Erfindungsgemäße, hypoallergene Varianten, DNA-Moleküle und rekombinanten Expressionsvektoren beziehungsweise erfindungsgemäße Arzneimittel können insbesondere zur Prophylaxe und/oder zur Behandlung von Krankheiten und Krankheitszuständen verwendet werden. Erfindungsgemäße Arzneimittel eignen sich besonders zur Behandlung und/oder Prophylaxe von Typ 1-Allergien, das heißt zur spezifischen Immuntherapie (Hyposensibilisierung) von Patienten mit Gräserpollenallergie oder zur präventiven Immuntherapie von Gräserpollenallergien an deren Auslösung Gruppe 5-Allergene von Spezies der *Poaceae* beteiligt sind. Erfindungsgemäße DNA-Moleküle und rekombinanten Expressionsvektoren können zur entsprechenden immuntherapeutischen und -prophylaktischen DNA-Vakzinierung eingesetzt werden.

Gegenstand der Erfindung ist auch die Verwendung wenigstens einer erfindungsgemäßen, hypoallergenen Variante zur Herstellung eines Arzneimittels zur Prävention und/oder therapeutischen Behandlung von Typ-1 Allergien, an deren Auslösung Gruppe 5-Allergene der Süßgräser ursächlich beteiligt sind.

Erfindungsgemäß ist auch die Verwendung wenigstens eines erfindungsgemäßen DNA-Moleküls und/oder eines erfindungsgemäßen, rekombinanten Expressionsvektors einschließlich deren Mischungen in allen Verhältnissen zur Herstellung eines Arzneimittels zur immuntherapeutischen DNA-Vakzinierung.

Ein weiterer Gegenstand der Erfindung ist eine pharmazeutische Zubereitung, enthaltend wenigstens eine erfindungsgemäße, hypoallergene Variante, ein erfindungsgemäßes DNA-Molekül und/oder einen erfindungsgemäßen, rekombinanten Expressionsvektor, einschließlich deren Mischungen in allen Verhältnissen und gegebenenfalls weitere Träger- und/oder Hilfsstoffe zur Prävention und/oder therapeutischen Behandlung von Typ-1 Allergien.

Insbesondere eigenen sich erfindungsgemäße, pharmazeutische Zubereitungen zur Prävention und/oder therapeutischen Behandlung von Typ-1 Allergien, an deren Auslösung Gruppe 5-Allergene der Süßgräser ursächlich beteiligt sind.

Pharmazeutische Zubereitungen im Sinne dieser Erfindung können als Therapeutika in der Human- oder Veterinärmedizin verwendet werden und demnach an Menschen und Tiere, insbesondere Säugetiere wie Affen, Hunde, Katzen, Ratten oder Mäuse verabreicht und bei der therapeutischen Behandlung des menschlichen oder tierischen Körpers sowie bei der Bekämpfung der oben aufgeführten Krankheiten verwendet werden. Sie können weiterhin als Diagnostika oder als Reagenzien Verwendung finden.

Bei Verwendung von erfindungsgemäßen Zubereitungen oder Arzneimittel werden die erfindungsgemäßen hypoallergenen Varianten, DNA-Moleküle oder rekombinanten Expressionsvektoren in der Regel analog zu bekannten, käuflich erhältlichen Zubereitungen oder Präparaten verwendet, vorzugsweise in Dosierungen zwischen 0,001 und 500 mg, bei hypoallergenen Varianten etwa 1-500 µg, bevorzugt 5-200 µg pro Dosis in der Erhaltungsphase. Die Zubereitung kann ein- oder mehrmals pro Tag verabreicht werden, z.B. zwei-, drei- oder viermal am Tag. Typischerweise werden die Dosen in einer Aufdosierungsphase bis zur Erhaltungsdosis gesteigert. Hierfür sind verschiedene Schemata der Aufdosierung und Erhaltung möglich. Diese können beispielsweise bei der subkutanen Immuntherapie (SCIT) Kurzzeittherapien (begrenzte Anzahl von Injektionen vor Beginn der saisonalen Beschwerden, typischerweise 4 - 7 Injektionen), präsaisonale Therapien (Beginn der Therapie vor der Pollensaison, typischerweise mit wöchentlichen Injektionen während der Steigerungsphase und monatlichen Injektionen mit der Erhaltungsdosis bis zum Beginn der Pollensaison) oder ganzjährige Therapien (Aufdosierungsphase typischerweise mit bis zu 16 wöchentlichen Injektionen gefolgt von monatlichen Injektionen mit der Erhaltungsdosis, bei Bedarf reduzierte Dosis während der Pollensaison) umfassen. Bei der sublingualen Immuntherapie mit wässrigen, oder festen Präparaten (Tabletten, Wafer etc.) kann die Einleitung der Therapie mit oder ohne Aufdosierungsphase stattfinden. Die Therapie erfolgt bevorzugt mit täglichen Dosen ganzjährig, kann aber auch präsaisonal oder mit anderen Applikationsschemata (z.B. jeden zweiten Tag, wöchentlich, monatlich) durchgeführt werden.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder angestrebt wird.

Darüber hinaus bedeutet der Ausdruck "therapeutisch wirksame Menge" eine Menge, die, verglichen zu einem entsprechenden Subjekt, das diese Menge nicht erhalten hat, folgendes zur Folge hat: verbesserte Heilbehandlung, Heilung, Prävention oder Beseitigung einer Krankheit, eines Krankheitsbildes, eines Krankheitszustandes, eines Leidens, einer Störung oder Verhinderung von Nebenwirkungen oder auch die Verminderung des Fortschreitens einer Krankheit, eines Leidens oder einer Störung. Die Bezeichnung "therapeutisch wirksame Menge" umfasst auch die Mengen, die wirkungsvoll sind, die normale physiologische Funktion zu erhöhen.

Arzneimittel lassen sich zur Verabreichung über einen beliebigen geeigneten Weg, beispielsweise auf oralem (einschließlich buccalem bzw. sublingualem), rektalem, pulmonalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Arzneimittel können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

Zur parenteralen Anwendung dienen insbesondere Lösungen, vorzugsweise ölige oder wässrige Lösungen, ferner Suspensionen, Emulsionen oder Implantate. Die erfindungsgemäßen Allergenvarianten können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen und/oder mehrere weitere Wirkstoffe enthalten. Weiterhin können durch entsprechende Formulierung der erfindungsgemäßen Allergenvarianten Depotpräparate, beispielsweise durch Adsorption an Aluminiumhydroxid, Kalziumphosphat oder Tyrosin erhalten werden.

Als Trägerstoffe kommen organische oder anorganische Substanzen in Frage, die sich für die parenterale Applikation eignen und mit erfindungsgemäßen Gruppe 5-Allergenvarianten nicht reagieren. Beispiele hierfür sind Trägerstoffe wie Wasser, pflanzliche Öle, Benzylalkohole, Polyethylenglykole, Glycerintriacetat, Gelatine, Kohlenhydrate, wie Lactose oder Stärke, Magnesiumstearat, Talk, Lanolin oder Vaseline.

Zur Verabreichung der erfindungsgemäßen Arzneimittel eignet sich vorzugsweise die parenterale Applikation. Im Falle der parenteralen Applikation sind die intravenöse, subkutane, intradermale oder intralymphatische Applikation besonders bevorzugt. Im Falle der intravenösen Applikation kann die Injektion direkt oder auch als Zusatz zu Infusionslösungen erfolgen.

Zu den an die parenterale Verabreichung angepassten Arzneimitteln gehören wässrige und nichtwässrige sterile Injektionslösungen, die Antioxidantien, Puffer, Bakteriostatika und Lösungsvermittler enthalten, durch die die Formulierung isotonisch mit dem Blut des zu behandelnden Empfängers gemacht wird, enthalten, sowie wässrige und nichtwässrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so dass nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Falls erwünscht, können erfindungsgemäße Zubereitungen oder Medikamente einen oder mehrere weitere Wirkstoffe und/oder einen oder mehrere Wirkverstärker (Ajuvantien) enthalten.

Ein weiterer Gegenstand der vorliegenden Erfindung sind somit pharmazeutische Zubereitungen, enthaltend weitere Wirk- und/oder Hilfsstoffe. Ein bevorzugter Gegenstand der Erfindung sind erfindungsgemäße pharmazeutische Zubereitungen, dadurch gekennzeichnet, dass es sich bei den weiteren Wirkstoffen um Allergene oder Varianten davon handelt. Beispiele für geeignete weitere Wirkstoffe sind andere Allergene, insbesondere Allergene der Süßgräser, besonders bevorzugt Allergene aus der Unterfamilie der *Pooideae,* bevorzugt aus den Gruppen *Poodae* und *Triticodae,* bevorzugt vertreten durch *Phleum pratense, Holcus Ianatus*, *Phalaris aquatica, Dactylis glomerata, Lolium perenne, Poa pratensis, Hordeum vulgare, Secale cereale* und *Triticum aestivum,* beispielsweise Gruppe 1-, 2-, 3-, 4-, 5-, 6-, 7-, 10-, 12- oder 13-Allergene und Varianten davon, beispielsweise hypoallergene Varianten, Fragmente, Multimere, Hybridmoleküle oder rekombinanten Fusionsproteine. Ein weiterer Gegenstand der vorliegenden Erfindung sind pharmazeutische Zubereitungen, enthaltend wenigstens einen weiteren Hilfsstoff, besonders bevorzugt sogenannte Wirkverstärker. Beispiele für Wirkverstärker sind Aluminiumhydroxid, Monophosphoryl-Lipid A, Aktivatoren von Toll-like Rezeptoren wie z.B. Lipopolysaccharide und CpG-Oligonukleotide, Vitamin D3, mycobakterielle Antigene und Moleküle aus Parasiten (z.B. Schistosomen oder Filarien), wie z.B. Cystatin oder ES-62.

Ein Gegenstand der Erfindung sind auch Sets (Kits) bestehend aus getrennten Packungen von
a) einer erfindungsgemäßen pharmazeutischen Zubereitung, enthaltend eine wirksame Menge einer erfindungsgemäßen, hypoallergenen Variante, DNA-Moleküls oder rekombinanten Expressionsvektors
b) einer pharmazeutischen Zubereitung, enthaltend eine wirksamen Menge eines weiteren pharmazeutischen Wirkstoffs und/oder Wirkverstärkers.

Das Set enthält geeignete Behälter, wie Schachteln oder Kartons, individuelle Flaschen, Beutel oder Ampullen. Das Set kann z.B. separate Ampullen enthalten, in denen jeweils eine erfindungsgemäße Formulierung, enthaltend eine wirksame Menge einer erfindungsgemäßen, hypoallergenen Variante, eines DNA-Moleküls oder eines rekombinanten Expressionsvektors und einer Formulierung eines weiteren Arzneimittelwirkstoffs in gelöster oder in lyophylisierter Form vorliegt.

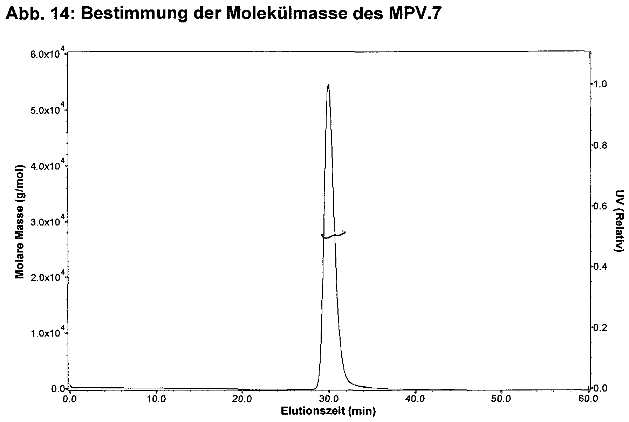

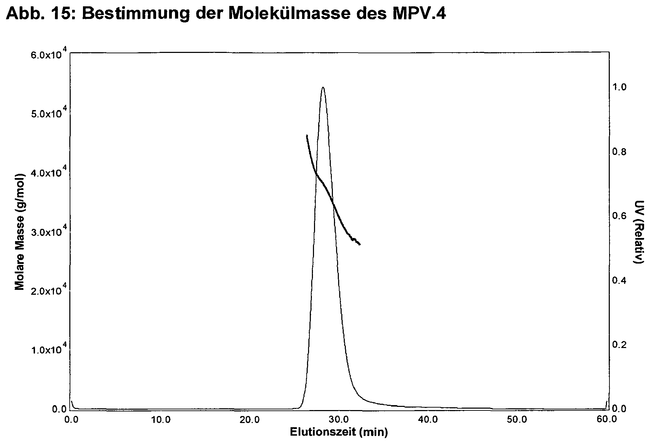

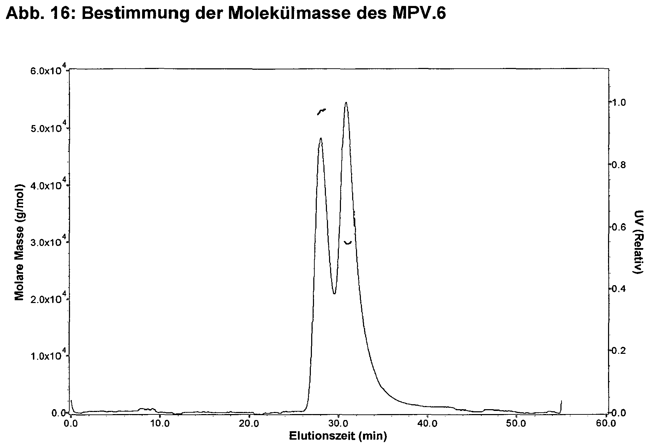

Erläuterungen der Abbbildungen:
**Abb. 1a****: Alignment deduzierter Aminosäuresequenzen der Gruppe 5-Allergene der Poaceae**: Alignment von reifen Gruppe 5 Sequenzen verschiedener Spezies. Die Boxen zeigen die Lage der α-Helices von Phl p 6 (PDB-Eintrag 1 NLX) und der C-terminalen Hälfte eines Phl p 5.02-Fragmentes (PDB-Eintrag 1 L3P) an. Die Aminosäurepositionen der Prolinreste sind entsprechend ihrer Position im reifen Phl p 5.0109 beschriftet.
   Sequenzreferenzen: Phl p 5.0101 (Phleum pratense IUIS-Sequenz,
   UniProtKB Q40960), Phl p 5.0104 (Phleum pratense IUIS-Sequenz,
   UniProtKB P93467), Phl p 5.0109 (Phleum pratense IUIS-Sequenz,
   UniProtKB Q84UI2), Phl p 5.0201 (Phleum pratense IUIS-Sequenz,
   UniProtKB Q40963), Phl p 6.0101 (Phleum pratense IUIS-Sequenz,
   UniProtKB P43215), Lol p 5.0101 (Lolium perenne IUIS-Sequenz, UniProtKB Q40237), Pha a 5.0101 (Phalaris aquatica IUIS-Sequenz, UniProtKB P56164), Dac g 5 (Dactylis glomerata, UniProtKB Q93XD9), Hol l 5.0101 (Holcus lanatus IUIS-Sequenz, UniProtKB 023972), Hol l 5.0201 (Holcus lanatus IUIS-Sequenz, UniProtKB 23971), Poa p 5.0101 (Poa pratensis IUIS-Sequenz, UniProtKB Q9FPR0), Tri a 5 (Triticum aestivum, UniProtKB Q70JP9), Hor v 5 (Hordeum vulgare, EST TC190653).
**Abb. 1b****: Konservierung der Prolinreste**
**Abb. 2****: Arbeitsmodelle zur Lage der Prolinreste in der 3D-Struktur des Phl p 5a**
   3D-Homologie-Modelle des N-terminalen (Aminosäuren 31-139 des Phl p 5.0109; Vorlagemolekül: Phl p 6, PDB-Eintrag 1 NLX; dargestellt auf linker Seite) und C-terminalen 4-Helix-Bündels (Aminosäuren 155-255; Vorlagemolekül: Phl p 5b-Fragment, PDB-Eintrag 1 L3P; rechts).
   a. Stark vereinfachte Modelle der beiden 4-Helix-Bündel. H1-H8: Helices 1-8. Proline sind mit ihrer Positionsbezeichnung und Atomstruktur gezeigt. Die Prolinreste P146 und P256 liegen in Bereichen, die aufgrund fehlender Strukturdaten in den 3D-Modellen der Vorlagemoleküle nicht dargestellt werden konnten (gepunktete Linien). Die zwischen Helix 2 und 3 dargestellte Schleife ist spekulativ, da das Vorlagemolekül Phl p 6 keine zu diesem Phl p 5a-Bereich homologe Region besitzt.
   b. Oberflächenmodelle. Alle darstellbaren Prolinreste sind an der Oberfläche exponiert (schwarz eingefärbt).
**Abb. 3****: rPhl p 5a wt (IUIS Eintrag Phl p 5.0109); cDNA-Sequenz (GenBank Eintrag: AJ555152; 855 bp), SEQ ID NO:1**
**Abb. 4****: rPhl p 5a wt (IUIS Eintrag Phl p 5.0109); Deduzierte Aminosäuresequenz (UniProtKB Eintrag: Q84UI2; 284 aa), SEQ ID NO:2**
**Abb. 5****: N-terminaler Histidin-Fusionsanteil; DNA-Sequenz (57 bp), SEQ ID NO:3**
**Abb. 6****: N-terminaler Histidin-Fusionsanteil; Aminosäuresequenz (19 aa) SEQ ID:4**
**Abb. 7****: Ergebnisse der IgE-Inhibitonstests mit Phl p 5a-Varianten mit Prolin-Deletionen in einzelnen Schleifen bei Einsatz eines Serum-Pools**
   (a) + (b): Daten aus je einem Einzelexperiment mit Doppelbestimmung. Die Symbole repräsentieren die Mittelwerte der Duplikate bei Messung von je acht Konzentrationen der Inhibitoren. Die waagrechten Linien der Fehlerbalken zeigen die Einzelwerte der Doppelbestimmung.
   (c): Zusammenfassung der Ergebnisse mehrerer Einzelexperimente.
      Linker Balken (dunkelgrau): IgE-Inhibition relativ zu rPhl p 5a wt bei 5 µg/ml Inhibitorkonzentration.
      Rechter Balken (hellgrau): IgE-Inhibition relativ zu rPhl p 5a wt bei 1,25 µg/ml Inhibitorkonzentration.
      Die Balkenhöhe zeigt den Mittelwert an, der aus einer Anzahl (n) von Einzelexperimenten erhoben wurde. Die Fehlerbalken zeigen maximale (waagrechte Linie des oberen Fehlerbalkens) und minimale (waagrechte Linie des unteren Fehlerbalkens) Werte, die in einer Anzahl (n) ausgewerteter Einzelexperimente erhoben wurden.
      Feste Phase: rPhl p 5a wt. Serum-Pool: Bor 18/100, Allergopharma.
**Abb. 8****: Vergleich der IgE-Inhibition von rPhl p 5a d[P117,180] + 6His mit den Einzelstellen-Prolinmutanten rPhl p 5a d[P117] + 6His und d[P180] + 6His**
   Zusammenfassung der Ergebnisse mehrerer Einzelexperimente:
      Linker Balken (dunkelgrau): IgE-Inhibition relativ zu rPhl p 5a wt bei 5 µg/ml Inhibitorkonzentration.
      Rechter Balken (hellgrau): IgE-Inhibition relativ zu rPhl p 5a wt bei 1,25 µg/ml Inhibitorkonzentration.
      Die Balkenhöhe zeigt den Mittelwert an, der aus einer Anzahl (n) von Einzelexperimenten erhoben wurde. Die Fehlerbalken zeigen maximale (waagrechte Linie des oberen Fehlerbalkens) und minimale (waagrechte Linie des unteren Fehlerbalkens) Werte, die in einer Anzahl (n) ausgewerteter Einzelexperimente erhoben wurden.
      Feste Phase: rPhl p 5a wt. Serum-Pool: Bor 18/100, Allergopharma.
**Abb. 9****: Vergleich der IgE-Inhibition von MPV.3 + 6His mit den Einzelstellen-Prolinmutanten rPhl p 5a d[P57, 58] + 6His und d[P229] + 6His**
   Zusammenfassung der Ergebnisse mehrerer Einzelexperimente:
      Linker Balken (dunkelgrau): IgE-Inhibition relativ zu rPhl p 5a wt bei 5 µg/ml Inhibitorkonzentration.
      Rechter Balken (hellgrau): IgE-Inhibition relativ zu rPhl p 5a wt bei 1,25 µg/ml Inhibitorkonzentration.
      Die Balkenhöhe zeigt den Mittelwert an, der aus einer Anzahl (n) von Einzelexperimenten erhoben wurde.
      Die Fehlerbalken zeigen maximale (waagrechte Linie des oberen Fehlerbalkens) und minimale (waagrechte Linie des unteren Fehlerbalkens) Werte, die in einer Anzahl (n) ausgewerteter Einzelexperimente erhoben wurden.
      Feste Phase: rPhl p 5a wt. Serum-Pool: Bor 18/100, Allergopharma.
**Abb. 10****: Vergleich der IgE-Inhibition von MPV.3 + 6His mit den Einzelstellen-Prolinmutanten rPhl p 5a d[P211] + 6His, P211 L + 6His, K61 E + 6His und E205K + 6His**
   Zusammenfassung der Ergebnisse mehrerer Einzelexperimente:
      Linker Balken (dunkelgrau): IgE-Inhibition relativ zu rPhl p 5a wt bei 5 µg/ml Inhibitorkonzentration.
      Rechter Balken (hellgrau): IgE-Inhibition relativ zu rPhl p 5a wt bei 1,25 µg/ml Inhibitorkonzentration.
      Die Balkenhöhe zeigt den Mittelwert an, der aus einer Anzahl (n) von Einzelexperimenten erhoben wurde.
      Die Fehlerbalken zeigen maximale (waagrechte Linie des oberen Fehlerbalkens) und minimale (waagrechte Linie des unteren Fehlerbalkens) Werte, die in einer Anzahl (n) ausgewerteter Einzelexperimente erhoben wurden.
      Feste Phase: rPhl p 5a wt. Serum-Pool: Bor 18/100, Allergopharma.
**Abb. 11****: Test auf funktionelle Allergenität des MPV.3 + 6His**
   Nachweis der reduzierten funktionellen Allergenität von MPV.3 + 6His mittels Basophilen-Aktivierungstest mit Basophilen von vier klinisch definierten Gräserpollen-Allergikern (P).
   Waagrechte Linie: Niveau der Stimulans durch eine Negativkontrolle.
**Abb. 12****: IgE-Inhibitonstest mit MPV.4 + 6His und MPV.4**
   Linker Balken (dunkelgrau): IgE-Inhibition relativ zu rPhl p 5a wt bei 5 µg/ml Inhibitorkonzentration.
   Rechter Balken (hellgrau): IgE-Inhibition relativ zu rPhl p 5a wt bei 1,25 µg/ml Inhibitorkonzentration.
   Die Balkenhöhe zeigt den Mittelwert an, der aus einer Anzahl (n) von Einzelexperimenten erhoben wurde.
   Die Fehlerbalken zeigen maximale (waagrechte Linie des oberen Fehlerbalkens) und minimale (waagrechte Linie des unteren Fehlerbalkens) Werte, die in einer Anzahl (n) ausgewerteter Einzelexperimente erhoben wurden.
   Feste Phase: rPhl p 5a wt. Serum-Pool: Bor 18/100, Allergopharma.

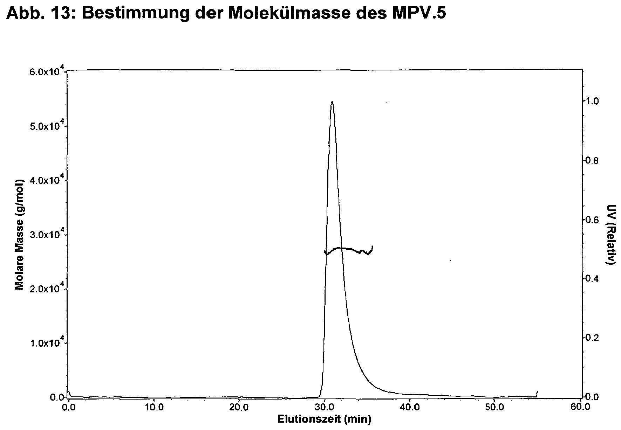

**Abb. 13****: Bestimmung der Molekülmasse des MPV.5**
   Chromatogramm einer analytischen SEC mit Online-Molekulargewichtsbestimmung.
   Dargestellt ist das relative UV-Signal bei 280 nm (rechte Y-Achse) sowie das Molekulargewicht (linke Y-Achse; Messpunktlinie im Peakbereich), aufgetragen gegen die Elutionszeit (X-Achse).
   Zur Online-Bestimmung der Proteinkonzentration wurde der Brechungsindex-Detektor OptilabrEX (Wyatt, Santa Barbara, USA) eingesetzt. Die Lichtstreuung der Partikel wurde mit dem Mehrwinkeldetektor MiniDAWN Treos (Wyatt) bestimmt. Die Berechnung der Partikelmasse erfolgte mit der Software ASTRA 5.3.2.17 (Wyatt) über Debeye-Formalismus mit einem Brechungsindexinkrement von 0,180 ml/g.
   Säule: Superdex 200 GL 10/300 (GE Healthcare, Uppsala, Schweden). Der Größenausschluss (*t*₀) liegt bei 20,45 min (entspricht ∼670 kD).
   Laufmittel: 20 mM Tris 8,0, 150 mM NaCl
**Abb. 14****: Bestimmung der Molekülmasse des MPV.7**
   Chromatogramm einer analytischen SEC mit Online-Molekulargewichtsbestimmung.
   Dargestellt ist das relative UV-Signal bei 280 nm (rechte Y-Achse) sowie das Molekulargewicht (linke Y-Achse; Messpunktlinie im Peakbereich), aufgetragen gegen die Elutionszeit (X-Achse).
   Zur Online-Bestimmung der Proteinkonzentration wurde der Brechungsindex-Detektor OptilabrEX (Wyatt, Santa Barbara, USA) eingesetzt. Die Lichtstreuung der Partikel wurde mit dem Mehrwinkeldetektor MiniDAWN Treos (Wyatt) bestimmt. Die Berechnung der Partikelmasse erfolgte mit der Software ASTRA 5.3.2.17 (Wyatt) über Debeye-Formalismus mit einem Brechungsindexinkrement von 0,180 ml/g.
   Säule: Superdex 200 GL 10/300 (GE Healthcare, Uppsala, Schweden). Der Größenausschluss (*t*₀) liegt bei 20,45 min (entspricht ∼670 kD).
   Laufmittel: 20 mM Tris 8,0, 150 mM NaCl
**Abb. 15****: Bestimmung der Molekülmasse des MPV.4**
   Chromatogramm einer analytischen SEC mit Online-Molekulargewichtsbestimmung.
   Dargestellt ist das relative UV-Signal bei 280 nm (rechte Y-Achse) sowie das Molekulargewicht (linke Y-Achse; Messpunktlinie im Peakbereich), aufgetragen gegen die Elutionszeit (X-Achse).
   Zur Online-Bestimmung der Proteinkonzentration wurde der Brechungsindex-Detektor OptilabrEX (Wyatt, Santa Barbara, USA) eingesetzt. Die Lichtstreuung der Partikel wurde mit dem Mehrwinkeldetektor MiniDAWN Treos (Wyatt) bestimmt. Die Berechnung der Partikelmasse erfolgte mit der Software ASTRA 5.3.2.17 (Wyatt) über Debeye-Formalismus mit einem Brechungsindexinkrement von 0,180 ml/g.
   Säule: Superdex 200 GL 10/300 (GE Healthcare, Uppsala, Schweden). Der Größenausschluss (*t*₀) liegt bei 20,45 min (entspricht ∼670 kD).
   Laufmittel: 20 mM Tris 8,0, 150 mM NaCl
**Abb. 16****: Bestimmung der Molekülmasse des MPV.6**
   Chromatogramm einer analytischen SEC mit Online-Molekulargewichtsbestimmung.
   Dargestellt ist das relative UV-Signal bei 280 nm (rechte Y-Achse) sowie das Molekulargewicht (linke Y-Achse; Messpunktlinie im Peakbereich), aufgetragen gegen die Elutionszeit (X-Achse).
   Zur Online-Bestimmung der Proteinkonzentration wurde der Brechungsindex-Detektor OptilabrEX (Wyatt, Santa Barbara, USA) eingesetzt. Die Lichtstreuung der Partikel wurde mit dem Mehrwinkeldetektor MiniDAWN Treos (Wyatt) bestimmt. Die Berechnung der Partikelmasse erfolgte mit der Software ASTRA 5.3.2.17 (Wyatt) über Debeye-Formalismus mit einem Brechungsindexinkrement von 0,180 ml/g.
   Säule: Superdex 200 GL 10/300 (GE Healthcare, Uppsala, Schweden). Der Größenausschluss (*t*₀) liegt bei 20,45 min (entspricht ∼670 kD).
   Laufmittel: 20 mM Tris 8,0, 150 mM NaCl
**Abb. 17****: IgE-Bindung an immobilisiertes MPV.4, MPV.5, MPV.6 und MPV.7**
   Test auf Bindung von IgE an auf Nitrocellulose-Membranstreifen
   immobilisierte Phl p 5-Proteine.
   rPhl p 5b wt: Rekombinante Phl p 5b-Isoform
   rPhl p 5a wt: Rekombinante Phl p 5a-Isoform
   nPhl p 5a/b: Phl p 5-Allergen natürlichen Ursprungs, bestehend aus Protein der a- und b-Isoform (Allergopharma)
   MPV.4: d[P57, P58, P117, P146, P155, P180, P229] K61E E205K P211L
   MPV.5: d[P57, P58, P117, P180, P229] K61E E205K P211L
   MPV.6: d[P57, P58, P117, P146, P155, P180, P229] P211 L
   MPV.7: d[P57, P58, P117, P180, P229] P211 L
   HSA: Albumin aus Humanserum (Negativkontrolle).
   Total: Kontrolle für gleichmäßige Beladung der Streifen. Färbung mit Reagenz DB71 (Sigma, USA)
   SP: Serum-Pool Bor18/100 (Allergopharma)
**Abb. 18****: IgE-Inhibitonstest mit MPV.5 und MPV.4**
   Linker Balken (dunkelgrau): IgE-Inhibition relativ zu rPhl p 5a wt bei 5 µg/ml Inhibitorkonzentration.
   Rechter Balken (hellgrau): IgE-Inhibition relativ zu rPhl p 5a wt bei 1,25 µg/ml Inhibitorkonzentration.
   Die Balkenhöhe zeigt den Mittelwert an, der aus einer Anzahl (n) von Einzelexperimenten erhoben wurde.
   Die Fehlerbalken zeigen maximale (waagrechte Linie des oberen Fehlerbalkens) und minimale (waagrechte Linie des unteren Fehlerbalkens) Werte, die in einer Anzahl (n) ausgewerteter Einzelexperimente erhoben wurden.
   Feste Phase: rPhl p 5a wt. Serum-Pool: Bor 18/100, Allergopharma.
**Abb. 19****: IgE-Inhibitonstest mit MPV.6 und MPV.7**
   Linker Balken (dunkelgrau): IgE-Inhibition relativ zu rPhl p 5a wt bei 5 µg/ml Inhibitorkonzentration.
   Rechter Balken (hellgrau): IgE-Inhibition relativ zu rPhl p 5a wt bei 1,25 µg/ml Inhibitorkonzentration.
   Die Balkenhöhe zeigt den Mittelwert an, der aus einer Anzahl (n) von Einzelexperimenten erhoben wurde.
   Die Fehlerbalken zeigen maximale (waagrechte Linie des oberen Fehlerbalkens) und minimale (waagrechte Linie des unteren Fehlerbalkens) Werte, die in einer Anzahl (n) ausgewerteter Einzelexperimente erhoben wurden.
   Feste Phase: rPhl p 5a wt. Serum-Pool: Bor 18/100, Allergopharma.
**Abb. 20****: Test auf funktionelle Allergenität des MPV.4**
   Nachweis der reduzierten funktionellen Allergenität von MPV.4 mittels Basophilen-Aktivierungstest mit Basophilen von vier klinisch definierten Gräserpollen-Allergikern (P).
   Waagrechte Linie: Niveau der Stimulans durch eine Negativkontrolle.

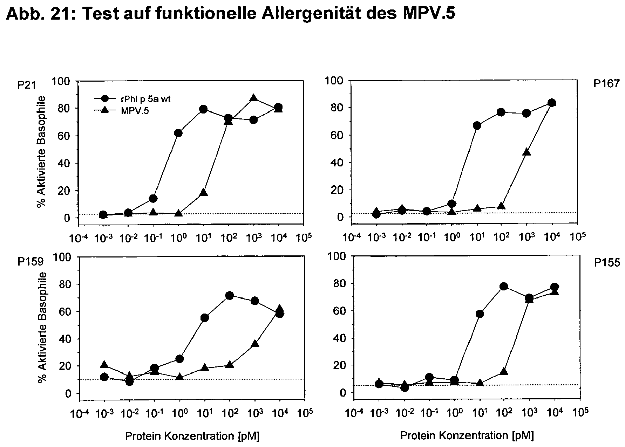

**Abb. 21****: Test auf funktionelle Allergenität des MPV.5**
   Nachweis der reduzierten funktionellen Allergenität von MPV.5 mittels Basophilen-Aktivierungstest mit Basophilen von vier klinisch definierten Gräserpollen-Allergikern (P).
   Waagrechte Linie: Niveau der Stimulans durch eine Negativkontrolle.

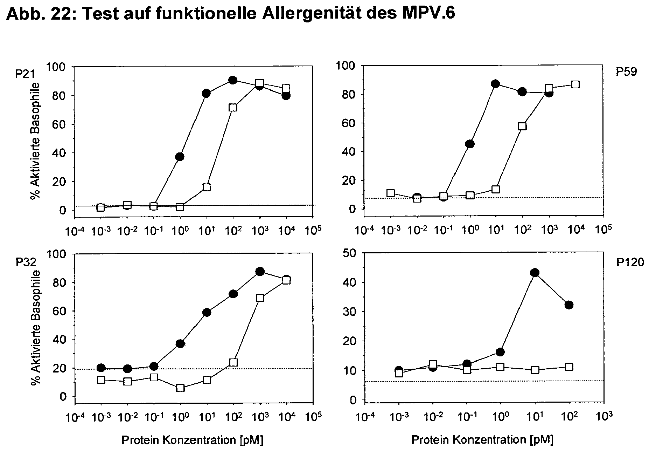

**Abb. 22****: Test auf funktionelle Allergenität des MPV.6**
   Nachweis der reduzierten funktionellen Allergenität von MPV.6 mittels Basophilen-Aktivierungstest mit Basophilen von klinisch definierten Gräserpollen-Allergikern (P).
   Waagrechte Linie: Niveau der Stimulans durch eine Negativkontrolle.

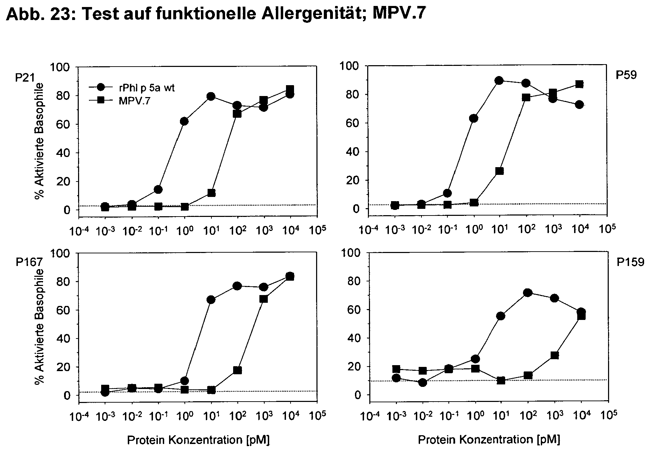

**Abb. 23****: Test auf funktionelle Allergenität; MPV.7**
   Nachweis der reduzierten funktionellen Allergenität von MPV.7 mittels Basophilen-Aktivierungstest mit Basophilen von vier klinisch definierten Gräserpollen-Allergikern (P).
   Waagrechte Linie: Niveau der Stimulans durch eine Negativkontrolle.
**Abb. 24****: rPhl p 5b wt precursor (IUIS Eintrag Phl p 5.0201); Deduzierte Aminosäuresequenz 284 aa (Swiss-Prot: Q40963.2), SEQ ID NO:5**
   Auch ohne weitere Ausführungsformen wird davon ausgegangen, dass ein Fachmann die obige Beschreibung im weitesten Umfang nutzen kann. Die bevorzugten Ausführungsformen sind deshalb lediglich als beschreibende, keineswegs aber als in irgendeiner Weise limitierende Offenbarung aufzufassen.

Die folgenden Beispiele sollen somit die Erfindung erläutern, ohne sie zu begrenzen. Sofern nichts anderes angegeben ist, bedeuten Prozentangaben Gewichtsprozent. Alle Temperaturen sind in Grad Celsius angegeben. "Übliche Aufarbeitung": Man gibt, falls erforderlich, Wasser hinzu, stellt, falls erforderlich, je nach Konstitution des Endprodukts auf pH-Werte zwischen 2 und 10 ein.

Die folgenden erfindungsgemäßen, hypoallergenen Varianten wurden biotechnologisch hergestellt und charakterisiert. Die Herstellung und Charakterisierung der Substanzen ist jedoch auch auf anderen Wegen für den Fachmann durchführbar. Beispielsweise können die erfindungsgemäßen, hypoallergenen Varianten auch chemisch synthetisiert werden. Die nachfolgend beschriebenen, erfindungsgemäßen, hypoallergennen Varianten sind ebenfalls Gegenstand der Erfindung.

### Beispiel 1: Varianten des Phl p 5a mit einer oder zwei benachbarten Prolindeletionen

Die Herstellung der Varianten rPhl p 5a d[P57, P58] + 6His, rPhl p 5a d[P85] + 6His, rPhl p 5a d[P117] + 6His, rPhl p 5a d[P146, P155] + 6His, rPhl p 5a d[P180] + 6His, rPhl p 5a d[P211] + 6His, rPhl p 5a d[P229] + 6His und rPhl p 5a d[P256] + 6His sowie deren immunologische Charakterisierung ist im Folgenden dargestellt. Analog wird das rekombinante unveränderte Allergen (rPhl p 5 wt + 6His) hergestellt und untersucht und analog können auch die hypoallergenen Varianten der weiteren erfindungsgemäßen Gruppe 5-Allergene der Süßgräser sowie deren Wildtyp-Proteine, insbesondere Lol p 5 und Poa p 5, hergestellt und untersucht werden.

### Gentechnische Konstruktion:

Die Synthese der DNA der Varianten erfolgt durch die Verbindung langer überlappender DNA-Oligonukleotide und der Amplifizierung der DNA durch ein PCR-Standardverfahren. Die Kodons werden so gewählt, dass die deduzierte Aminosäuresequenz auf der des Phl p 5.0109 basiert (Abb. 3, 4). Die Mutationen für die Prolin-Deletionen werden eingeführt indem spezifische Oligonukleotide in den PCR-Reaktionen eingesetzt werden, denen die entsprechenden Kodons für Prolin fehlen. Die Oligonukleotide werden so gewählt, dass das deduzierte Protein am 5'-Ende einen Hexa-Histidin-Fusionsanteil trägt (Abb. 5, 6). Die cDNAs werden in den Expressionsvektor pTrcHis2 Topo (Invitrogen, Carlsbad, USA) ligiert. Die Richtigkeit der DNA wird durch Sequenzierung bestätigt.

### Expression, Reinigung und Biochemische Analytik:

Die Expression erfolgt in *Escherichia coli* (Stamm Top10; Invitrogen). rPhl p 5a wt sowie die Varianten werden durch die spezifische Bindung der N-terminalen Histidinreste an eine Ni2+-Chelat-Matrix (Immobilized-Metal-Ion-Affinity-Chromatography, IMAC; Material: HiTrap, GE Healthcare, Uppsala, Schweden) gereinigt. Die Abwesendheit unlöslicher Proteinaggregate wird durch UV-Vis-Spektroskopie bestätigt.

### Nachweis der reduzierten IgE-Bindung:

Die Untersuchung der IgE-Bindungsfähigkeit der Testsubstanzen erfolgt mit einem EAST-IgE-Inhibitionstest (Enzyme Allergosorbent Test). Bei diesem Verfahren kann die Allergen/IgE-Interaktion in Lösung untersucht werden, womit störende Maskierungen von Epitopen der Testsubstanz etwa durch Immobilisierung an eine Membran ausgeschlossen werden kann.

Der EAST-Hemmtest wird wie folgt ausgeführt. Mikrotiterplatten werden mit den Allergenen, hier rekombinantes Wildtyp-Phl p 5.0109 (rPhl p 5a wt), beschichtet. Nach Entfernung der nicht gebundenen Allergenmoleküle durch Waschen wird die Platte zur Vermeidung späterer unspezifischer Bindungen mit Rinderserum-Albumin blockiert. IgE-Antikörper von Allergikern werden in Form eines repräsentativen Pools von Einzelallergikerseren (Serum-Pool) in geeigneter Verdünnung mit den Allergen-beschichteten Mikrotiterplatten inkubiert. Die Menge der allergengebundenen IgE-Antikörper wird über ein Anti-human-IgE/Alkalische Phosphatase-Konjugat durch die Umsetzung eines Substrates zu einem farbigen Endprodukt photometrisch quantifiziert.

Die Bindung der IgE-Antikörper wird durch ein lösliches Allergen bzw. die zu prüfende Substanz (rekombinantes modifiziertes Allergen) in Abhängigkeit von der Konzentration substanzspezifisch gehemmt. Die in Abb. 7. dargestellten Ergebnisse von IgE-Inhibitionstests mit den rekombinanten Allergenvarianten des Phl p 5a zeigen, dass durch Deletion der Prolinreste P57 und P58; P117; P146 und P155; P180; P211 oder P229, nicht jedoch durch Deletion von P85 oder P256 eine verminderte IgE-Bindungsfähigkeit des Phl p 5a verursacht wird. Eine niedrigere Hemmwirkung deutet auf einen Verlust von IgE-Epitopen hin.

### Beispiel 2: Varianten des Phl p 5a mit Kombinationen von Prolindeletionen

Die Herstellung und immunologische Charakterisierung der Variante rPhl p 5a d[P117, P180] + 6His ist im Folgenden beispielhaft für erfindungsgemäße, hypoallergene Varianten der Gruppe-5-Allergene der Poaceae mit Kombinationen von mehreren Prolin-Deletionen entsprechend den Aminosäurepositionen des 57 und 58, 117, 146, und 155, 180, 211 und 229 bezogen auf Phl p 5.0109 dargestellt. Analog wird das rekombinante unveränderte Allergen (rPhl p 5 wt + 6His) hergestellt und untersucht und analog können auch die hypoallergenen Varianten der weiteren erfindungsgemäßen Gruppe 5-Allergene der Süßgräser sowie deren Wildtyp-Proteine, insbesondere Lol p 5 und Poa p 5, hergestellt und untersucht werden.

### Gentechnische Konstruktion:

Die Synthese der Varianten erfolge durch die Verbindung langer überlappender DNA- Oligonukleotide und der Amplifizierung der DNA durch PCR. Die Kodons werden so gewählt, dass die deduzierte Aminosäuresequenz auf der des Phl p 5.0109 basiert. Die Mutationen für die Prolin-Deletionen werden eingeführt, indem spezifische Oligonukleotide in den PCR-Reaktionen eingesetzt werden, denen die entsprechenden Kodons für Prolin fehlen. Die Oligonukleotide werden so gewählt, dass das deduzierte Protein am 5'-Ende einen Hexa-Histidin-Fusionsanteil trägt. Die cDNAs werden in den Expressionsvektor pTrcHis2 Topo (Invitrogen) und in Escherichia coli transformiert. Die Richtigkeit der DNA wird durch Sequenzierung bestätigt.

### Expression, Reinigung und Biochemische Analytik:

Die Expression erfolgt in *Escherichia coli* (Stamm Top10; Invitrogen). Die Varianten werden durch IMAC gereinigt. Die Reinheit der eluierten Proteine wird durch SDS-PAGE kontrolliert und die Abwesendheit unlöslicher Proteinaggregate durch UV-Vis-Spektroskopie bestätigt.

### Nachweis der Reduzierten IgE-Bindung:

Die Untersuchung der IgE-Bindungsfähigkeit der Testsubstanzen erfolgt mit einem EAST-Hemmtest mit IgE-Antikörpern von Allergikern, die in Form eines repräsentativen Serum-Pools eingesetzt wurden. Die in Abb. 8 dargestellten Ergebnisse zeigen, dass IgE-Inhibition der Variante rPhl p 5a d[P117, P180] + 6His deutlich geringer ist als die der Varianten mit nur einer der Prolin-Deletionen rPhl p 5a d[P117] + 6His und rPhl p 5a d[P180] + 6His. Dieses Ergebnis zeigt, dass die Kombination von einzelnen Prolin-Deletionen zu einer erhöhten Reduktion der IgE-Bindungsfähigkeit des Phl p 5a führen kann.

### Beispiel 3: Hypoallergene Variante rPhl p 5a d[P57, P58, P85, P117, P146, P155, P180, P211, P229, P256] (MPV.1 + 6His)

Die Allergenvariante MPV.1 + 6His weist eine Kombination der Deletionen aller untersuchter Prolinreste des Phl 5a auf. Das Ziel der Herstellung ist eine maximal reduzierte IgE-Bindungsfähigkeit bei akzeptabler Proteinlöslichkeit.

### Gentechnische Konstruktion:

Die Synthese der Varianten erfolgt durch die Verbindung langer überlappender DNA- Oligonukleotide und der Amplifizierung der DNA durch PCR. Die Kodons werden so gewählt, dass die deduzierte Aminosäuresequenz auf der des Phl p 5.0109 basiert. Die Mutationen für die Prolin-Deletionen werden eingeführt, indem spezifische Oligonukleotide in den PCR-Reaktionen eingesetzt eurden, denen die entsprechenden Kodons für Prolin fehlen.

Die Oligonukleotide werden so gewählt, dass das deduzierte Protein am 5'-Ende einen Hexa-Histidin-Fusionsanteil trägt. Die cDNA werden in den Expressionsvektor pTrcHis2 Topo (Invitrogen) und in Escherichia coli transformiert. Die Richtigkeit der DNA wird durch Sequenzierung bestätigt.

### Expression:

Die Expression erfolgt in *Escherichia coli* (Stamm Top10; Invitrogen). Die Proteine werden von der Wirtszelle als unlösliche *Inclusion Bodies* abgelegt.

### Test der Löslichkeit:

Die Proteinaggregate werden nach Zellaufschluss mit einer Standardmethode in einer Reinheit von ca. 80% isoliert und durch Behandlung mit einer 6 Molaren Lösung Guanidiniumhydrochlorid denaturierend solubilisiert.

Die denaturierten Proteine werden für diese Testung bei 4°C 1:50 in einem Volumen von 25 ml verdünnt und über Nacht bei 4°C gehalten. Am Folgetag wird eine eventuell sichtbare Präzipitatbildung organoleptisch überprüft. Nach einer Ankonzentrierung werden die Proben zentrifugiert und die klaren Überstände mittels UV-Vis-Spektroskopie auf die Anwesendheit von unlöslichen Mikro-Aggregaten hin untersucht.

Die systematische Untersuchung zum Löslichkeitsverhalten der Variante MPV.1 + 6His zeigt, dass einen anschließende Überführung der Proteine in eine Guanidiniumhydrochlorid-freie Formulierung stets mit der Bildung von Proteinaggregaten einhergeht und somit nicht möglich ist (Tab. 1)

### Beispiel 4: Hypoallergene Variante rPhl p 5a d[P57, P58, P117, P146, P155, P180, P211, P229] (MPV.2 + 6His)

Die Herstellung und immunologische Charakterisierung der Variante MPV.2+ 6His ist im Folgenden beispielhaft für hypoallergene Varianten der Gruppe-5-Allergene der Poaceae mit Kombinationen von Prolin-Deletionen entsprechend den Aminosäurepositionen 57, 58, 117, 146, 155, 180, 211 und 229 bezogen auf Phl p 5.0109 dargestellt. Analog wird das rekombinante unveränderte Allergen (rPhl p 5 wt + 6His) hergestellt und untersucht und analog können auch die hypoallergenen Varianten der weiteren erfindungsgemäßen Gruppe 5-Allergene der Süßgräser sowie deren Wildtyp-Proteine, insbesondere Lol p 5 und Poa p 5, hergestellt und untersucht werden.

### Gentechnische Konstruktion:

Die Synthese der Varianten erfolgt durch die Verbindung langer überlappender DNA- Oligonukleotide und der Amplifizierung der DNA durch PCR. Die Kodons werden so gewählt, dass die deduzierte Aminosäuresequenz auf der des Phl p 5.0109 basiert.

Die Mutationen für die Prolin-Deletionen werden eingeführt indem spezifische Oligonukleotide in den PCR-Reaktionen eingesetzt werden, denen die entsprechenden Kodons für Prolin fehlen.

Die Oligonukleotide werden so gewählt, dass das deduzierte Protein am 5'-Ende einen Hexa-Histidin-Fusionsanteil trägt. Die cDNA werden in den Expressionsvektor pTrcHis2 Topo (Invitrogen) ligiert und in Escherichia coli transformiert. Die Richtigkeit der DNA wird durch Sequenzierung bestätigt.

### Expression:

Die Expression erfolgt in *Escherichia coli* (Stamm Top10; Invitrogen). Die Proteine werden von der Wirtszelle als unlösliche *Inclusion Bodies* abgelegt.

### Test der Löslichkeit:

MPV.2 + 6His zeigt eine schlechte Löslichkeit im gesamten untersuchten pH-Bereich von 4,5-9,0. Lediglich mit einer Lösungen, die Tween 80 und L-Argininmonohydrochlorid enthält, kann die Bildung visuell sichtbarer Präzipitate verhindert werden, jedoch werden nachfolgend auch in diesem Ansatz Mikro-Aggregate nachgewiesen (Tab. 2).

### Beispiel 5: Hypoallergene Variante rPhl p 5a d[P57, P58, P229] K61 E, E205K, P211 L (MPV.3 + 6His)

Die Herstellung und immunologische Charakterisierung der Variante MPV.3 + 6His ist im Folgenden beispielhaft für hypoallergene Varianten der Gruppe-5-Allergene der Poaceae mit Kombinationen von Deletionen der Prolinreste 57, 58, 117, 146, 155, 180 oder 229 entsprechend der Aminosäurepositionen des Phl p 5 5.0109 dargestellt, bei denen der Prolinrest 211 in eine beliebige andere Aminosäure mutiert wird oder bei denen zusätzlich Lysin 61 in Glutamat oder Glutamat 205 in Lysin umgewandelt wird. Analog wird das rekombinante unveränderte Allergen (rPhl p 5 wt + 6His) hergestellt und untersucht und analog können auch die hypoallergenen Varianten der weiteren erfindungsgemäßen Gruppe 5-Allergene der Süßgräser sowie deren Wildtyp-Proteine, insbesondere Lol p 5 und Poa p 5, hergestellt und untersucht werden.

Die Auswirkung der Mutationen K61 E, E205K und P211 L, die in der Aminosäuresequenz des MPV.3 +6His enthalten sind, auf die IgE-Bindungsfähigkeit wird untersucht indem die Varianten rPhl p 5a K61 E + 6His, rPhl p 5a E205K + 6His und rPhl p 5a P211 L + 6His hergestellt und immunologisch charakterisiert werden.

### Gentechnische Konstruktion:

Die für MPV.3 + 6His kodierende Nukleotidsequenz wird in diesem Beispiel überraschend durch mehrere Polymerasefehler bei einer cDNA-Synthese generiert. Die DNA wird in den Expressionsvektor pTrcHis2 Topo (Invitrogen) ligiert. Die DNA kodierend für die Varianten rPhl p 5a K61E + 6His, rPhl p 5a E205K + 6His und rPhl p 5a P211 L + 6His werden gezielt hergestellt und in den Expressionsvektor pTrcHis2 Topo ligiert. Die Richtigkeit der generierten cDNA-Sequenzen wird durch DNA-Sequenzierung überprüft.

### Expression :

Die Expression erfolgt in *Escherichia coli* (Stamm Top10; Invitrogen). Die Proteine werden von der Wirtszelle als unlösliche *Inclusion Bodies* abgelegt.

### Test der Löslichkeit von MPV.3 + 6His:

Die Proteinaggregate werden nach Zellaufschluss in einer Reinheit von ca. 80% isoliert und durch Behandlung mit einer 6 Molaren Lösung Guanidiniumhydrochlorids denaturierend solubilisiert. Die Reihentestung verschiedener Lösungen zur Überprüfung der Löslichkeitseigenschaften von MPV.3 + 6His in nicht-denaturierender Umgebung wird analog zu den Experimenten mit den Varianten MPV.1 + 6His und MPV.2 + 6His durchgeführt.

Es wird überraschend festgestellt, dass die Variante MPV.3 + 6His während des Verfahrens der Verdünnung eine hohe Löslichkeit bei leicht basischem pH aufweist (Tab. 3). Dieses Verhalten steht im Gegensatz zu den zuvor untersuchten Varianten MPV.1 + 6His und MPV.2 + 6His und stellt einen entscheidenden Vorteil für die Darstellung des Proteins in einer nicht-denaturierenden Umgebung dar.

Reinigung von MPV.3 + 6His, sowie den Mutanten rPhl p 5a K61 E + 6His, rPhl p 5a E205K + 6His und rPhl p 5a P211 L + 6His:
Die denaturierten Proteine werden 1:50 in 20 mM Tris, 150 mM NaCl, pH 9,0 verdünnt und über Nacht bei 8°C gehalten. Die chromatographische Reinigung erfolgt durch IMAC (HiTrap-Material, GE Healthcare) und SEC (Superdex 75-Material, GE Healthcare). Abschließend liegen die Proteine stabil und löslich in 25 mM Natriumphosphat-Puffer mit 150 mM NaCl, pH 7,5 vor.

### Biochemische Analytik:

Die Reinheit wird durch SDS-PAGE kontrolliert. Die Abwesendheit unlöslicher Proteinaggregate wird durch UV-Vis-Spektroskopie bestätigt.

### Nachweis der reduzierten IgE-Bindung von MPV.3 + 6His:

Die Untersuchung der IgE-Bindungsfähigkeit von MPV.3 + 6His erfolge mit einem EAST-Hemmtest mit IgE-Antikörpern von Allergikern, die in Form eines repräsentativen Serum-Pools eingesetzt werden. Die in Abb. 9 dargestellten Ergebnisse zeigen, dass die IgE-Inhibition von MPV.3 + 6His deutlich geringer ist, als die der Varianten rPhl p 5a d[P57, P58] + 6His und rPhl p 5a d[P229] + 6His.

Zur weiteren Charakterisierung der Mutationen K61 E, E205K und P211 L der Variante MPV.3 + 6His werden die drei Varianten rPhl p 5a K61 E + 6His, rPhl p 5a E205K + 6His und rPhl p 5a P211 L + 6His durch EAST untersucht. Die Variante rPhl p 5a P211L+ 6His zeigt ein vergleichbar niedriges Hemmverhalten als die zuvor getestete Punktmutante rPhl p 5a d[P211] + 6His. Die Varianten rPhl p 5a K61 E + 6His und rPhl p 5a E205K+ 6His zeigen eine leicht erniedrigte IgE-Bindung (Abb. 10).

Es kann gefolgert werden, dass die drei Punktmutationen K61 E, E205K und P211 L zur deutlich erniedrigten IgE-Bindung von MPV.3 + 6His beitragen.

Nachweis der Reduktion der Funktionellen Allergenität von MPV.3 + 6His: Die funktionelle Wirkung von MPV.3 + 6His bei der Vernetzung von membrangebundenem IgE der Effektorzellen und deren Aktivierung wird in vitro untersucht.

Für den Basophilen-Aktivierungstest wird heparinisiertes Vollblut von Grasspollen-Allergikern mit verschiedenen Konzentrationen der Testsubstanzen inkubiert. Dabei können allergene Substanzen spezifische IgE-Antikörper, welche mit den hochaffinen IgE-Rezeptoren der basophilen Granulozyten assoziert sind, binden. Die durch die Allergenmoleküle ausgelöste Vernetzung der IgE/Rezeptor-Komplexe führt zu einer Signaltransduktion, die in der Degranulation der Effektorzellen und somit dem Auslösen der allergischen Reaktionen in vivo resultiert.

Die Allergen-induzierte Aktivierung von basophilen Immunozyten kann *in vitro* durch Quantifizierung der Expression eines mit der Signaltransduktion der IgE-Rezeptor-Vernetzung gekoppelten Oberflächenproteins (CD203c) nachgewiesen werden (Kahlert et al., Clinical Immunology and Allergy in Medicine Proceedings of the EAACI 2002 (2003) Naples, Italy 739-744). Die Zahl der exprimierten Oberflächenproteine auf einer Zelle und der Prozentwert der aktivierten Zellen eines Zellpools wird über die Bindung eines fluoreszensmarkierten monoklonalen Antikörpers an das Oberflächenprotein und anschließende Analyse durch Fluoreszensaktivierte Durchflusszytometrie hochsensitiv gemessen.

MPV.3 + 6His zeigt hier eine reduzierte Aktivierung von basophilen Granulozyten von Gräserpollenallergikern im Vergleich zu rPhl p 5a wt und somit eine funktionell reduzierte Allergenität (Abb. 11)

### Beispiel 6: Hypoallergene Variante rPhl p 5a d[P57, P58, 117, 146, 155, 180, P229] K61 E, E205K, P211 L (MPV.4)

Die Herstellung und immunologische Charakterisierung der Variante MPV.4 ist im Folgenden beispielhaft für hypoallergene Varianten der Gruppe-5-Allergene der Poaceae mit Kombinationen von Deletionen der Prolinreste 57, 58, 117, 146, 155, 180 oder 229 entsprechend der Aminosäurepositionen des Phl p 5.0109) dargestellt, bei denen der Prolinrest 211 in eine beliebige andere Aminosäure mutiert wird oder bei denen zusätzlich Lysin 61 in Glutamat oder Glutamat 205 in Lysin umgewandelt wird. Analog wird das rekombinante unveränderte Allergen (rPhl p 5 wt + 6His) hergestellt und untersucht und analog können auch die hypoallergenen Varianten der weiteren erfindungsgemäßen Gruppe 5-Allergene der Süßgräser sowie deren Wildtyp-Proteine, insbesondere Lol p 5 und Poa p 5, hergestellt und untersucht werden.

### Gentechnische Konstruktion von MPV.4 (+ 6His):

Zur Herstellung der cDNA des Histidin-Fusionsproteins von MPV.4 (MPV.4 + 6His) wird ein Fragment der bereits klonierten cDNA von MPV.1 + 6His in das bereits vorhandene Plasmid MPV.3 + 6His/ pTrcHis2 Topo umkloniert. Zur Herstellung des Fusionsanteil-freien Proteins (MPV.4) wird die DNA ohne den für den Histidin-Fusionsanteil kodierenden Abschnitt in den Vektor pTMP (Allergopharma, Reinbek) ligiert. Die Richtigkeit der Sequenz wird durch DNA-Sequenzierung überprüft.

### Expression von MPV.4 (+ 6His):

Die Expression erfolgt entweder als Histidin-Fusionsprotein (Expressionsvektor pTrcHis2Topo; Invitrogen) in *Escherichia coli* (Stamm Top10; Invitrogen) oder ohne Fusionsanteil (Expressionsvektor pTMP) in *Escherichia coli* (Stamm BL21; Merck, Darmstadt). In beiden Fällen werden die rekombinanten Proteine als *Inclusion Bodies* abgelegt. Die Proteine werden mit einer 6 molaren Lösung Guanidiniumhydrochlorid solubilisiert.

### Test der Löslichkeit von MPV.4 + 6His:

Die Reihentestung verschiedener Lösungen zur Überprüfung der Löslichkeitseigenschaften von MPV.4 + 6His in nicht-denaturierender Umgebung wird analog zu den Experimenten mit der Varianten MPV.1 + 6His durchgeführt. Es wird festgestellt, dass die Variante MPV.4 + 6His Löslichkeit bei leicht basischem pH aufweist (Tab. 4)

### Reinigung von MPV.4 + 6His:

Das Fusionsprotein wird im präparativen Maßstab durch IMAC (HiTrap-Material, GE Healthcare) und SEC (Superdex 75-Material, GE Healthcare) gereinigt und liegt abschließend in 25 mM Natriumphosphat-Puffer mit 150 mM NaCl, pH 7,5 vor.

### Reinigung des Nicht-Fusionsproteins MPV.4:

Die Lösung des denaturierten Nicht-Fusionsproteins werden analog zu dem Reinigungsschema des Fusionsproteins zunächst mit 20 mM Tris, 150 mM NaCl, pH 9,0 1:50 verdünnt und über Nacht bei 4° gehalten. In einem ersten Reinigungsschritt wwird das Zielprotein durch Hydrophobe-Interaktions-Chromatography (HIC) angereichert.

Proteine binden bei hoher Ionenstärke an eine HIC-Säule in Abhängigkeit ihrer Oberflächenhydophobizität mit spezifischer Stärke, so dass sie durch graduelle Verringerung der Salzkonzentration selektiv eluiert werden können.

Die verdünnte Proteinlösung wird zunächst 1:2 mit 20 mM Tris, 2 M Ammoniumsulfat, 150 mM NaCl, pH 9,0 weiter verdünnt, um eine finale Ammoniumsulfat-Konzentration von 1 M zu erzielen. Diese Proteinlösung wird dann über eine HiTrap Butyl-S FF HIC-Säule (GE Healthcare, Uppsala, Schweden) gegeben und nach Bindung des Zielproteins mit 20 mM Tris, 150 mM NaCl, pH 9,0 graduell eluiert.

Die HIC-Eluate werden als Vorbereitung für den zweiten Reinigungsschritt über eine Sephadex-G25-Säule (GE Healthcare) in 20 mM Tris, 50 mM NaCl, pH 8,0 umgepuffert.

Als zweiten Reinigungsschritt werden die Eluate auf eine Anionenaustausch-Chromatographie-Säule (HiTrap Q HP, GE Healthcare) aufgetragen und der Durchlauf aufgefangen. Das Zielprotein befindet sich dabei im Durchlauf, während Fremdproteine an die Säule binden und effektiv abgereinigt werden.

Bei dem AIEX-Verfahren werden Proteine mit negativer Oberflächenladung bei geringer Ionenstärke an das Säulenmaterial gebunden, während ungeladene oder positiv geladene Proteine nicht binden.

Im letzten Schritt wird der AIEX-Durchlauf über eine SEC-Säule (Superdex 75, GE Healthcare) nachgereinigt, sodass das Zielprotein abschließend in 20 mM Tris, 150 mM NaCl, pH 8,0 vorliegt.

### Biochemische Analytik von MPV.4 (+ 6His):

Die Reinheit der Proteine wird durch SDS-PAGE kontrolliert. Die Identität des Fusionsanteil-freien MPV.4 wird durch Bestimmung der molekularen Masse mittels Massenspektroskopie (MALDI-TOF) und Sequenzierung der N-Terminalen Aminosäuren bewiesen (Sequenz: A-D-L-G-Y-G-P-A-T) (Tab. 6). Die Abwesendheit unlöslicher Proteinaggregate wwird durch UV-Vis-Spektroskopie bestätigt.

Die Molekulargewichts-Analyse von MPV.4 mittels SEC/MALS/RI ergibt überraschend, dass die Masse der eluierten Proteine zwischen 28,2 und 49,3 kD liegt (mittlere Masse 36,9 kD). Da die theoretische Masse des Monomers bei 27,6 kD liegt, ist davon auszugehen, dass ein Gemisch aus Monomeren und Dimeren vorliegt (Abb. 15, Tab. 6). Das qualitativ gleiche Ergebnis wird mit dem Fusionsprotein erhalten.

### Nachweis der reduzierten IgE-Bindung von MPV.4 (+ 6His):

Die Untersuchung der IgE-Bindungsfähigkeit erfolgt mit einem EAST-Hemmtest mit IgE-Antikörpern von Allergikern, die in Form eines repräsentativen Serum-Pools eingesetzt werden.

Die in Abb. 12 dargestellten Ergebnisse zeigen, dass MPV.4 sowohl mit als auch ohne Fusionsanteil eine gleich stark verminderte IgE-Bindung aufweist. Es ist somit gezeigt, dass die reduzierte IgE-Reaktivität nicht von dem Vorhandensein des Histidin-tags abhängt.

Eine einfache Testmethode zur Bestimmung der IgE-Reaktivität von spezifischem IgE aus Allergikerseren an membrangebundene Testproteine ist der Streifentest. Dafür werden die Testsubstanzen in gleicher Konzentration und Menge nebeneinander an einen Streifen von Nitrocellulose-Membran unter nicht-denaturierenden Bedingungen gebunden. Eine Reihe solcher Membranstreifen kann parallel mit unterschiedlichen Allergikerseren inkubiert werden. Nach einem Waschschritt werden die spezifisch gebundenen IgE-Antikörper durch eine Farbreaktion, vermittelt von einem Anti-human IgE/Alkalische Phosphatase-Konjugat, auf der Membran sichtbar gemacht.

Die Ergebnisse der Variante MPV.4 unter Einsatz von individuellen Gräserpollen-Allergikerseren sind in Abb. 17 dargestellt. Es kommen Seren von Allergikern mit Antikörpern gegen natürliches Phl p 5 (nPhl p 5a/b, Gemisch aus Phl p 5a- und b-Isoform) zur Anwendung. Die IgE-Antikörper reagieren ebenso mit dem rekombinanten rPhl p 5a wt und dem ebenfalls untersuchten rekombinanten Wildtyp der b-Isoform (rPhl p 5b wt).

Es wird deutlich, dass die Phl p 5-spezifischen IgE-Antikörper aller Allergikerseren die Variante MPV.4 stark vermindert binden, während die rekombinanten Wildtyp-Proteine rPhl p 5a wt und rPhl p 5b wt genauso stark gebunden werden wie nPhl p 5a/b.

### Nachweis der Reduktion der Funktionellen Allergenität von MPV.4:

Die funktionelle Wirkung von MPV.4 bei der Vernetzung von membrangebundenem IgE der Effektorzellen und deren Aktivierung wird sowohl mit Fusionsproteinen als auch mit Nicht-Fusionsproteinen *in vitro* untersucht. Sowohl das Fusionsprotein als auch das Fusionsanteil freie MPV.4 zeigen hier eine stark reduzierte Aktivierung von basophilen Granulozyten von Gräserpollenallergikern im Vergleich zu rPhl p 5a wt und somit eine stark funktionell reduzierte Allergenität (Abb. 20).

### Beispiel 7: Hypoallergene Variante rPhl p 5a d[P57, P58, P117, P180, P229] K61 E, E205K, P211 L (MPV.5)

Die Herstellung und immunologische Charakterisierung der Variante MPV.5 ist im Folgenden beispielhaft für hypoallergene Varianten der Gruppe-5-Allergene der Poaceae mit Kombinationen von Deletionen der Prolinreste 57, 58, 117, 180 oder 229 entsprechend der Aminosäurepositionen des Phl p 5.0109 dargestellt, bei denen der Prolinrest 211 in eine beliebige andere Aminosäure mutiert wurd oder bei denen zusätzlich Lysin 61 in Glutamat oder Glutamat 205 in Lysin umgewandelt wird. Analog wird das rekombinante unveränderte Allergen (rPhl p 5 wt + 6His) hergestellt und untersucht und analog können auch die hypoallergenen Varianten der weiteren erfindungsgemäßen Gruppe 5-Allergene der Süßgräser sowie deren Wildtyp-Proteine, insbesondere Lol p 5 und Poa p 5, hergestellt und untersucht werden.

### Gentechnische Konstruktion von MPV.5 (+ 6His):

Zur Herstellung der cDNA des Histidin-Fusionsproteins (MPV.5 + 6His) wird ein Fragment der bereits klonierten cDNA von rPhl p 5a d[P117, P180] + 6His in das bereits vorhandene Plasmid MPV.3 + 6His/ pTrcHis2 Topo umkloniert. Zur Herstellung des Fusionsanteil-freien Proteins (MPV.5) wird die DNA ohne den für den Histidin-Fusionsanteil kodierenden Abschnitt in den Vektor pTMP (Allergopharma, Reinbek) ligiert. Die Richtigkeit der Sequenz wird durch DNA-Sequenzierung überprüft.

### Expression von MPV.5 (+ 6His):

Die Expression erfolge entweder als Histidin-Fusionsprotein (Expressionsvektor pTrcHis2Topo; Invitrogen) in *Escherichia coli* (Stamm Top10; Invitrogen) oder ohne Fusionsanteil (Expressionsvektor pTMP; Allergopharma) in *Escherichia coli* (Stamm BL21; Merck, Darmstadt).

In beiden Fällen werden die rekombinanten Proteine als *Inclusion Bodies* abgelegt. Die Proteine werden mittels einer 6 molaren Lösung Guanidiniumhydrochlorid solubilisiert.

### Reinigung von MPV.5 + 6His:

Das Fusionsprotein wird im präparativen Maßstab durch IMAC (HiTrap-Material, GE Healthcare) und SEC (Superdex 75-Material, GE Healthcare) gereinigt und liegt abschließend in 25 mM Natriumphosphat-Puffer mit 150 mM NaCl, pH 7,5 vor.

### Reinigung des Nicht-Fusionsproteins:

Die IB-Lösung des denaturierten Nicht-Fusionsproteins werden analog zu dem Reinigungsschema des Fusionsproteins zunächst mit 20 mM Tris, 150 mM NaCl, pH 9,0 1:50 verdünnt und über Nacht bei 4° gehalten.

In einem ersten Reinigungsschritt wuird das Zielprotein durch Hydrophobe-Interaktions-Chromatography (HIC) angereichert. Die verdünnte Proteinlösung wird zunächst 1:2 mit 20 mM Tris, 2 M Ammoniumsulfat, 150 mM NaCl, pH 9,0 weiter verdünnt, um eine finale Ammoniumsulfat-Konzentration von 1 M zu erzielen. Diese Proteinlösung wird dann über eine HiTrap Butyl-S FF HIC-Säule (GE Healthcare, Uppsala, Schweden) gegeben und nach Bindung des Zielproteins mit 20 mM Tris, 150 mM NaCl, pH 9,0 graduell eluiert.

Die HIC-Eluate werden als Vorbereitung für den zweiten Reinigungsschritt über eine Sephadex-G25-Säule (GE Healthcare) in 20 mM Tris, 50 mM NaCl, pH 8,0 umgepuffert. Als zweiten Reinigungsschritt werden die Eluate auf eine Anionenaustausch-Chromatographie-Säule (HiTrap Q HP, GE Healthcare) aufgetragen und der Durchlauf mit dem Zielprotein aufgefangen.

Im letzten Schritt wird der AIEX-Durchlauf über eine SEC-Säule (Superdex 75, GE Healthcare) nachgereinigt, sodass das Zielprotein abschließend in 20 mM Tris, 150 mM NaCl, pH 8,0 vorliegt.

### Biochemische Analytik von MPV.5 (+ 6His):

Die Reinheit der Proteine wird durch SDS-PAGE kontrolliert. Die Abwesendheit unlöslicher Proteinaggregate wird durch UV-Vis-Spektroskopie bestätigt. Die Identität des Fusionsanteil-freien MPV.5 wird durch Bestimmung der molekularen Masse mittels Massenspektroskopie (MALDI-TOF) und Sequenzierung der N-Terminalen Aminosäuresequenz bewiesen (Sequenz: A-D-L-G-Y-G-P-A-T) (Tab. 6).

Die Analyse von MPV.5 durch SEC/MALS/RI zeigt, dass das Protein ausschließlich als Monomer vorliegt (Abb. 13, Tab. 6). Ein vergleichbares Ergebnis wird mit dem Fusionsprotein erhalten. Die hohe Tendenz zur Dimerisierung, die bei der Variante MPV.4 festgestellt wird, ist somit vom Vorhandensein der Mutation d[P146, P155] abhängig.

### Nachweis der reduzierten IgE-Bindung von MPV.5 (+ 6His):

Die Untersuchung der IgE-Bindungsfähigkeit erfolgt mit einem EAST-Hemmtest mit IgE-Antikörpern von Allergikern, die in Form eines repräsentativen Serum-Pools eingesetzt werden. MPV.5 zeigt sowohl mit als auch ohne Fusionsanteil eine gleich stark verminderte IgE-Bindungsfähigkeit. Die IgE-Bindungsfähigkeit ist etwas höher als die der Variante MPV.4, was auf einer besseren Zugänglichkeit von IgE-Epitopen bei dem ausschliesslich monomer vorliegenden MPV.5 beruhen kann (Abb. 18).

Die Ergebnisse der Variante MPV.5 im Streifentest unter Einsatz von individuellen Gräserpolleri-Allergikerseren sind in Abb. 17 dargestellt. Es wird deutlich, dass die Phl p 5-spezifischen IgE-Antikörper aller Allergikerseren die Variante MPV.5 stark vermindert binden.

Nachweis der Reduktion der Funktionellen Allergenität von MPV.5 (+ 6His): Die funktionelle Wirkung von MPV.5 bei der Vernetzung von membrangebundenem IgE der Effektorzellen und deren Aktivierung wird sowohl mit Fusionsproteinen als auch mit Nicht-Fusionsproteinen in vitro untersucht. MPV.5 zeigt hier eine stark reduzierte Aktivierung von basophilen Granulozyten von Gräserpollenallergikern im Vergleich zu rPhl p 5a wt und somit eine stark funktionell reduzierte Allergenität (Abb. 21).

### Beispiel 8: Hypoallergene Variante rPhl p 5a d[P57, P58, P117, P146, P155, P180, P229] P211 L (MPV.6)

Die Herstellung und immunologische Charakterisierung der Variante MPV.6 ist im Folgenden beispielhaft für hypoallergene Varianten der Gruppe-5-Allergene der Poaceae mit Kombinationen von Deletionen der Prolinreste 57, 58, 117, 146, 155, 180 oder 229 entsprechend der Aminosäurepositionen des Phl p 5.0109 dargestellt, bei denen der Prolinrest 211 in eine beliebige andere Aminosäure mutiert wird. Analog wird das rekombinante unveränderte Allergen (rPhl p 5 wt + 6His) hergestellt und untersucht und analog können auch die hypoallergenen Varianten der weiteren erfindungsgemäßen Gruppe 5-Allergene der Süßgräser sowie deren Wildtyp-Proteine, insbesondere Lol p 5 und Poa p 5, hergestellt und untersucht werden.

### Gentechnische Konstruktion:

Zur Herstellung der DNA wird ein DNA-Fragment von MPV.2 + 6His in den zu diesem Zeitpunkt vorhandenen Vektor rPhl p 5a d[57, 58, 117, 180, 229] P211L/ pTMP (Allergopharma) ligiert. Die Richtigkeit der Sequenz wird durch DNA-Sequenzierung überprüft.

### Expression:

Die Expression erfolgt ausschließlich als Fusionsanteil-freies Protein in dem Expressionsvektor pTMP (Allergopharma) in *Escherichia coli* (Stamm BL21; Merck, Darmstadt). Die rekombinanten Proteine werden als *Inclusion Bodies* (IB) abgelegt.

### Reinigung:

Die IB-Lösung des denaturierten Nicht-Fusionsproteins wird zunächst mit 20 mM Tris, 150 mM NaCl, pH 9,0 1:50 verdünnt und über Nacht bei 4° gehalten. In einem ersten Reinigungsschritt wird das Zielprotein durch Hydrophobe-Interaktions-Chromatography (HIC) angereichert.

Die verdünnte Proteinlösung wird zunächst 1:2 mit 20 mM Tris, 2 M Ammoniumsulfat, 150 mM NaCl, pH 9,0 weiter verdünnt, um eine finale Ammoniumsulfat-Konzentration von 1 M zu erzielen. Diese Proteinlösung wird dann über eine HiTrap Butyl-S FF HIC-Säule (GE Healthcare, Uppsala, Schweden) gegeben und nach Bindung des Zielproteins mit 20 mM Tris, 150 mM NaCl, pH 9,0 graduell eluiert.

Die HIC-Eluate werden als Vorbereitung für den zweiten Reinigungsschritt über eine Sephadex-G25-Säule (GE Healthcare) in 20 mM Tris, 50 mM NaCl, pH 8,0 umgepuffert. Als zweiten Reinigungsschritt werden die Eluate auf eine Anionenaustausch-Chromatographie-Säule (HiTrap Q HP, GE Healthcare) aufgetragen und der Durchlauf mit dem Zielprotein aufgefangen. Im letzten Schritt wird der AIEX-Durchlauf über eine SEC-Säule (Superdex 75, GE Healthcare) nachgereinigt, sodass das Zielprotein abschließend in 20 mM Tris, 150 mM NaCl, pH 8,0 vorliegt.

### Biochemische Analytik:

Die Reinheit der Proteine wird durch SDS-PAGE kontrolliert. Die Identität des Fusionsanteil-freien MPV.6 wird durch Bestimmung der molekularen Masse mittels Massenspektroskopie (MALDI-TOF) und Sequenzierung der N-Terminalen Aminosäuresequenz bewiesen (Sequenz: A-D-L-G-Y-G-P-A-T) (Tab. 6).

Die Abwesendheit unlöslicher Proteinaggregate wird durch UV-Vis-Spektroskopie bestätigt. Bei der Analyse von MPV.6 mittels SEC/MALS/RI eluieren die gereinigten Proteine in zwei Peaks. Peak 1 repräsentiert die monomere und Peak 2 die dimere Form (Abb. 16; Tab. 6).

Die Dimerisierungsfähigkeit ist, wie auch anhand des Varianten MPV.4 und MPV.5 gezeigt wird, auf das Vorhandensein der Mutation d[P146, 155] zurückzuführen.

### Nachweis der reduzierten IgE-Bindung:

Die Untersuchung der IgE-Bindung erfolgt mit einem EAST-Hemmtest mit IgE-Antikörpern von Allergikern, die in Form eines repräsentativen Serum-Pools eingesetzt werden.Die in Abb. 19 dargestellten Ergebnisse zeigen, dass MPV.6 eine stark verminderte IgE-Bindung aufweist. Die Ergebnisse im Streifentest unter Einsatz von individuellen Gräserpollen-Allergikerseren sind in Abb. 17 dargestellt. Es wird deutlich, dass die Phl p 5-spezifischen IgE-Antikörper aller Allergikerseren die Variante MPV.6 stark vermindert binden.

### Nachweis der Reduktion der Funktionellen Allergenität:

Die funktionelle Wirkung von MPV.6 bei der Vernetzung von membrangebundenem IgE und der Aktivierung von basophilen Granulozyten ist im Vergleich zu rPhl p 5a wt stark reduziert, wie die Ergebnisse mit Vollblut von zwei Gräserpollenallergikern zeigen (Abb. 22).

### Beispiel 9: Hypoallergene Variante rPhl p 5a d[P57, P58, P117, P180, P229] P211 L (MPV.7)

Die Herstellung und immunologische Charakterisierung der Variante MPV.7 ist im Folgenden beispielhaft für hypoallergene Varianten der Gruppe-5-Allergene der Poaceae mit Kombinationen von Deletionen der Prolinreste 57, 58, 117, 180 oder 229 entsprechend der Aminosäurepositionen des Phl p 5a Wildtyps bzw. des Phl p 5.0109 dargestellt, bei denen der Prolinrest 211 in eine beliebige andere Aminosäure mutiert wird. Analog wird das rekombinante unveränderte Allergen (rPhl p 5 wt + 6His) hergestellt und untersucht und analog können auch die hypoallergenen Varianten der weiteren erfindungsgemäßen Gruppe 5-Allergene der Süßgräser sowie deren Wildtyp-Proteine, insbesondere Lol p 5 und Poa p 5, hergestellt und untersucht werden.

### Gentechnische Konstruktion:

Die Herstellung der DNA erfolgt, indem ein DNA-Fragment mittels spezifischer Oligonukleotide durch PCR-Verfahren hergestellt und dann in den zu diesem Zeitpunkt bereits vorhandenen Vektor rPhl p 5a d[P57, P58, P117, P180, P211, P229]/ pTMP (Allergopharma) umkloniert wird. Die Richtigkeit der Sequenz wird durch DNA-Sequenzierung überprüft.

### Expression:

Die Expression erfolgt als Fusionsanteil-freies Protein in dem Expressionsvektor pTMP (Allergopharma) in *Escherichia coli* (Stamm BL21; Merck, Darmstadt). Die rekombinanten Proteine werden als *Inclusion Bodies* (IB) abgelegt.

### Test der Löslichkeit:

Die Reihentestung zur Überprüfung der Löslichkeitseigenschaften des rekombinanten Proteins in nicht-denaturierender Umgebung zeigt eine hohe Löslichkeit des Proteins im leicht basischen pH-Bereich (Tab. 5).

### Reinigung:

Die IB-Lösung des denaturierten Nicht-Fusionsproteins wird zunächst mit 20 mM Tris, 150 mM NaCl, pH 9,0 1:50 verdünnt und über Nacht bei 4° gehalten. In einem ersten Reinigungsschritt wird das Zielprotein durch Hydrophobe-Interaktions-Chromatography (HIC) angereichert. Die verdünnte Proteinlösung wird zunächst 1:2 mit 20 mM Tris, 2 M Ammoniumsulfat, 150 mM NaCl, pH 9,0 weiter verdünnt, um eine finale Ammoniumsulfat-Konzentration von 1 M zu erzielen. Diese Proteinlösung wird dann über eine HiTrap Butyl-S FF HIC-Säule (GE Healthcare, Uppsala, Schweden) gegeben und nach Bindung des Zielproteins mit 20 mM Tris, 150 mM NaCl, pH 9,0 graduell eluiert.

Die HIC-Eluate werden als Vorbereitung für den zweiten Reinigungsschritt über eine Sephadex-G25-Säule (GE Healthcare) in 20 mM Tris, 50 mM NaCl, pH 8,0 umgepuffert. Als zweiten Reinigungsschritt werden die Eluate auf eine Anionenaustausch-Chromatographie-Säule (HiTrap Q HP, GE Healthcare) aufgetragen und der Durchlauf mit dem Zielprotein aufgefangen. Im letzten Schritt wird der AIEX-Durchlauf über eine SEC-Säule (Superdex 75, GE Healthcare) nachgereinigt, sodass das Zielprotein abschließend in 20 mM Tris, 150 mM NaCl, pH 8,0 vorliegt.

### Biochemische Analytik:

Die Reinheit der Proteine wird durch SDS-PAGE kontrolliert. Die Identität des Fusionsanteil-freien MPV.7 wird durch Bestimmung der molekularen Masse mittels Massenspektroskopie (MALDI-TOF) und Sequenzierung der N-Terminalen Aminosäuresequenz bewiesen (Sequenz: A-D-L-G-Y-G-P-A-T) (Tab. 6). Die Abwesendheit unlöslicher Proteinaggregate wird durch UV-Vis-Spektroskopie bestätigt. Die Analyse durch SEC/MALS/RI zeigt, dass die eluierten Proteine ausschließlich als Monomere vorliegen (Abb. 14; Tab. 6). Dies ist auf das Fehlen der Mutation d[P146, P155] zurückzuführen.

### Nachweis der reduzierten IgE-Bindung:

Die Untersuchung der IgE-Bindung erfolgt mit einem EAST-Hemmtest mit IgE-Antikörpern von Allergikern, die in Form eines repräsentativen Serum-Pools eingesetzt werden. Die in Abb. 19 dargestellten Ergebnisse zeigen, dass MPV.7 eine stark verminderte IgE-Bindungaufweist. Die Ergebnisse im Streifentest unter Einsatz von individuellen Gräserpollen-Allergikerseren sind in Abb. 17 dargestellt. Es wird deutlich, dass die Phl p 5-spezifischen IgE-Antikörper aller Allergikerseren die Variante MPV.7 stark vermindert binden.

### Nachweis der Reduktion der Funktionellen Allergenität:

Die funktionelle Wirkung von MPV.7 bei der Vernetzung von membrangebundenem IgE und der Aktivierung von basophilen Granulozyten von Gräserpollenallergikern ist im Vergleich zu rPhl p 5a wt stark reduziert (Abb. 23).

### T-Zell-Reaktivität der hypoallergenen Phl p 5a-Varianten:

Zur Untersuchung der T-Zell-Reaktivität werden oligoklonale T-Zell-Linien von Gräserpollen-Allergikern unter Stimulation mit natürlichenn nPhl p 5a- oder rPhl p 5a wt-Molekülen nach üblichen Verfahren etabliert. In einem Proliferationstest wurden die unterschiedlichen T-Zell-Linien mit dem Referenzallergen rPhl p 5a wt sowie den modifizierten rekombinanten Allergenvarianten stimuliert. Die Proliferationsrate wurde durch den Einbau von [3H]-Thymidin mit den üblichen Verfahren bestimmt.

Beispielhaft für die beschriebenen modifizierten Allergenvarianten sind hier die Ergebnisse der Proliferationstests von MPV.4 und MPV.7 mit T-Zell-Linien von 12 Gräserpollen-Allergikern dargestellt.

Die T-Zellreaktivität der Variante MPV.4, die die meisten Mutationen aller untersuchten Moleküle trägt, ist trotz der Veränderungen der Aminosäuresequenz im Vergleich zu dem unveränderten rPhl p 5a wt nicht erniedrigt, was den Erhalt entscheidender T-Zell-Epitope nachweist (Tab. 7).

Die Mutante MPV.7 enthält die geringste Zahl an veränderten Aminosäurepositionen aller untersuchten Multi-Prolinmutanten. Das Molekül stimuliert die humanen T-Lymphozyten erwartungsgemäß vergleichbar gut, wie das unveränderte Allergen rPhl p 5a (Tab. 8).

**Tab. 1: Löslichkeitsverhalten von MPV.1 + 6His**

| d[P57, 58] | | d[P85] | d[P117] | | d[146,155] | d[P180] | d[P211] | P211L | d[P229] | d[P256] | | K61E, E205K |
|---|---|---|---|---|---|---|---|---|---|---|---|---|
| x | | x | x | | x | x | x | | x | x | | |
| | | | | | | | | | | | | |
| **Lsg.** | **pH** | **NaCl** | | **Puffersubstanz** | | **Additiv** | | | **OK¹** | **A₂₈₀/A₃₃₀²** | **Bewertung³** | |
| **1** | 4,5 | 0,15 M | | 0,02 M Na-Acetat | | ohne | | | - | n.d. | - | |
| **2** | 5,5 | 0,15 M | | 0,02 M Na-Citrat | | ohne | | | - | n.d. | - | |
| **3** | 6,5 | 0,15 M | | 0,02 M Ka-Phosph. | | ohne | | | - | n.d. | - | |
| **4** | 7,5 | 0,15 M | | 0,02 M Na-Phosph. | | ohne | | | - | n.d. | - | |
| **5** | 8,0 | 0,15 M | | 0,02 M Tris | | ohne | | | - | n.d. | - | |
| **6** | 9,0 | 0,15 M | | 0,02 M Tris | | ohne | | | - | n.d. | - | |
| **7** | 8,0 | 0,075 M | | 0,02 M Tris | | 0,5 M L-Arginin-HCl; | | | + | 12,5 (-) | - | |
| | | | | | | 0,005% (w/v) Tween 80 | | | | | | |
| **8** | 8,0 | ohne | | 0,02 M Tris | | 0,005% (w/v) Tween 80 | | | + | 10,2 (-) | - | |
| **9** | 8,0 | ohne | | 0,02 M Tris | | 0,5 M L-Arginin-HCl | | | - | n.d. | - | |
| **10** | 7,5 | ohne | | 0,02 M Na-Phosph. | | 10% (w/v) Glyzerin | | | - | n.d. | - | |

| | | | | | | | | | | | | |
|---|---|---|---|---|---|---|---|---|---|---|---|---|
| ¹ Die *aus "Inclusion Bodies"* isolierten Proteine wurden zunächst mit 6 M Guanidiniumhydrochlorid denaturiert, anschließend 1:50 in einer nicht-denaturierenden Lösung (Lsg. 1-10) verdünnt und über Nacht bei 4°C gehalten. Am Folgetag erfolgte eine organoleptische Kontrolle (OK) bezüglich einer Trübung durch sichtbare Makro-Aggregate bzw. Präzipitate. (-) Trübung; (+) klare Lösung. ² UV/Vis-Spektralanalyse zum Nachweis von unlöslichen Mikro-Aggregaten in klaren Lösungen durch Bestimmung des Verhältnisses der Absorption bei 280 und 330 nm. Test der Ansätze nach Zentrifugation. A₂₈₀/A₃₃₀ ≤ 20: Präzipitation (-); A₂₈₀/A₃₃₀ ≤ 30: Präzipitationstendenz (o); A₂₈₀/A₃₃₀ > 30: keine Präzipitation (+). (n.d.) nicht durchgeführt. ³ Bewertung des Löslichkeitsverhaltens auf Basis der organoleptischen und spektrophotometrischen Analyse. (-) tendenziell unlöslich; (+) löslich. | | | | | | | | | | | | |

**Tab. 2: Löslichkeitsverhalten von MPV.2 + 6His**

| d[P57, 58] | | d[P85] | d[P117] | | d[146,155] | d[P180] | d[P211] | P211L | d[P229] | | d[P256] | | K61E, E205K |
|---|---|---|---|---|---|---|---|---|---|---|---|---|---|
| x | | | x | | x | x | x | | x | | | | |
| | | | | | | | | | | | | | |
| **Lsg.** | **pH** | **NaCl** | | **Puffersubstanz** | | **Additiv** | | | **OK¹** | **A₂₈₀/A₃₃₀²** | | **Bewertung³** | |
| **1** | 4,5 | 0,15 M | | 0,02 M Na-Acetal | | ohne | | | - | n.d. | | - | |
| **2** | 5,5 | 0,15 M | | 0,02 M Na-Citrat | | ohne | | | - | n.d. | | - | |
| **3** | 6,5 | 0,15 M | | 0,02 M Ka-Phosph. | | ohne | | | - | n.d. | | - | |
| **4** | 7,5 | 0,15 M | | 0,02 M Na-Phosph. | | ohne | | | - | n.d. | | - | |
| **5** | 8,0 | 0,15 M | | 0,02 M Tris | | ohne | | | - | n.d. | | - | |
| **6** | 9,0 | 0,15 M | | 0,02 M Tris | | ohne | | | - | n.d. | | - | |
| **7** | 8,0 | 0,075 M | | 0,02 M Tris | | 0,5 M L-Arginin-HCl; | | | + | 11,5(-) | | - | |
| | | | | | | 0,005% (w/v) Tween 80 | | | | | | | |
| **8** | 8,0 | ohne | | 0,02 M Tris | | 0,005% (w/v) Tween 80 | | | - | n.d. | | - | |
| **9** | 8,0 | ohne | | 0,02 M Tris | | 0,5 M L-Arginin-HCl | | | - | n.d. | | - | |
| **10** | 7,5 | ohne | | 0,02 M Na-Phosph. | | 10% (w/v) Glyzerin | | | - | n.d. | | - | |

| | | | | | | | | | | | | | |
|---|---|---|---|---|---|---|---|---|---|---|---|---|---|
| ¹ Die *aus "Inclusion Bodies"* isolierten Proteine wurden zunächst mit 6 M Guanidiniumhydrochlorid denaturiert, anschließend 1:50 in einer nicht-denaturierenden Lösung (Lsg. 1-10) verdünnt und über Nacht bei 4°C gehalten. Am Folgetag erfolgte eine organoleptische Kontrolle (OK) bezüglich einer Trübung durch sichtbare Makro-Aggregate bzw. Präzipitate. (-) Trübung; (+) klare Lösung. ² UV/Vis-Spektralanalyse zum Nachweis von unlöslichen Mikro-Aggregaten in klaren Lösungen durch Bestimmung des Verhältnisses der Absorption bei 280 und 330 nm. Test der Ansätze nach Zentrifugation. A₂₈₀/A₃₃₀ ≤ 20: Präzipitation (-); A₂₈₀/A₃₃₀ ≤ 30: Präzipitationstendenz (o); A₂₈o/A₃₃₀ > 30: keine Präzipitation (+). (n.d.) nicht durchgeführt. ³ Bewertung des Löslichkeitsverhaltens auf Basis der organoleptischen und spektrophotometrischen Analyse. (-) tendenziell unlöslich; (+) löslich. | | | | | | | | | | | | | |

**Tab. 3: Löslichkeitsverhalten von MPV.3 + 6His**

| d[P57, 58] | | d[P85] | d[P117] | | d[146,155] | d[P180] | d[P211] | | P211L | d[P22 9] | | d[P256] | | K61E, E205K |
|---|---|---|---|---|---|---|---|---|---|---|---|---|---|---|
| x | | | | | | | | | x | x | | | | x |
| | | | | | | | | | | | | | | |
| **Lsg.** | **pH** | **NaCl** | | **Puffersubstanz** | | **Additiv** | | | | **OK¹** | **A₂₈₀/A₃₃₀²** | | **Bewertung³** | |
| **1** | 4,5 | 0,15 M | | 0,02 M Na-Acetat | | ohne | | | | - | n.d. | | - | |
| **2** | 5,5 | 0,15 M | | 0,02 M Na-Citrat | | ohne | | | | - | n.d. | | - | |
| **3** | 6,5 | 0,15 M | | 0,02 M Ka-Phosph. | | ohne | | | | - | n.d. | | - | |
| **4** | 7,5 | 0,15 M | | 0,02 M Na-Phosph. | | ohne | | | | - | n.d. | | - | |
| **5** | 8,0 | 0,15 M | | 0,02 M Tris | | ohne | | | | + | 36,0 (+) | | + | |
| **6** | 9,0 | 0,15 M | | 0,02 M Tris | | ohne | | | | + | 42,6 (+) | | + | |
| **7** | 8,0 | 0,075 M | | 0,02 M Tris | | 0,5 M L-Arginin-HCl; | | | | + | 37,5 (+) | | + | |
| | | | | | | 0,005% (w/v) Tween 80 | | | | | | | | |
| **8** | 8,0 | ohne | | 0,02 M Tris | | 0,005% (w/v) Tween 80 | | | | + | 26,0 (o) | | - | |
| **9** | 8,0 | ohne | | 0,02 M Tris | | 0,5 M L-Arginin-HCl | | | | - | n.d. | | - | |
| **10** | 7,5 | ohne | | 0,02 M Na-Phosph. | | 10% (w/v) Glyzerin | | | | - | n.d. | | - | |

| | | | | | | | | | | | | | | |
|---|---|---|---|---|---|---|---|---|---|---|---|---|---|---|
| ¹ Die *aus "Inclusion Bodies"* isolierten Proteine wurden zunächst mit 6 M Guanidiniumhydrochlorid denaturiert, anschließend 1:50 in einer nicht-denaturierenden Lösung (Lsg. 1-10) verdünnt und über Nacht bei 4°C gehalten. Am Folgetag erfolgte eine organoleptische Kontrolle (OK) bezüglich einer Trübung durch sichtbare Makro-Aggregate bzw. Präzipitate. (-) Trübung; (+) klare Lösung. ² UV/Vis-Spektralanalyse zum Nachweis von unlöslichen Mikro-Aggregaten in klaren Lösungen durch Bestimmung des Verhältnisses der Absorption bei 280 und 330 nm. Test der Ansätze nach Zentrifugation. A₂₈₀/A₃₃₀ ≤ 20: Präzipitation (-); A₂₈₀/A₃₃₀ ≤ 30: Präzipitationstendenz (o); A₂₈o/A₃₃₀ > 30: keine Präzipitation (+). (n.d.) nicht durchgeführt. ³ Bewertung des Löslichkeitsverhaltens auf Basis der organoleptischen und spektrophotometrischen Analyse. (-) tendenziell unlöslich; (+) löslich. | | | | | | | | | | | | | | |

**Tab. 4: Löslichkeitsverhalten von MPV.4 + 6His**

| d[P57, 58] | | d[P85] | d[P117] | d[146,155] | d[P180] | d[P211] | P211L | d[P229] | | d[P256] | | K61E, E205K |
|---|---|---|---|---|---|---|---|---|---|---|---|---|
| x | | | x | x | x | | x | x | | | | x |
| | | | | | | | | | | | | |
| **Lsg.** | **pH** | **NaCl** | **Puffer substanz** | | **Additiv** | | | **OK¹** | **A₂₈₀/A₃₃₀²** | | **Bewertung³** | |
| **1** | 4,5 | 0,15 M | 0,02 M Na-Acetat | | ohne | | | - | n.d. | | - | |
| **2** | 5,5 | 0,15 M | 0,02 M Na-Citrat | | ohne | | | - | n.d. | | - | |
| **3** | 6,5 | 0,15 M | 0,02 M Ka-Phosph. | | ohne | | | - | n.d. | | - | |
| **4** | 7,5 | 0,15 M | 0,02 M Na-Phosph. | | ohne | | | + | 33,6 (+) | | + | |
| **5** | 8,0 | 0,15 M | 0,02 M Tris | | ohne | | | + | 34,3 (+) | | + | |
| **6** | 9,0 | 0,15 M | 0,02 M Tris | | ohne | | | + | 32,0 (+) | | + | |
| **7** | 8,0 | 0,075 M | 0.02 M Tris | | 0,5 M L-Arginin-HCl: | | | + | 34,8 (+) | | + | |
| | | | | | 0,005% (w/v) Tween 80 | | | | | | | |
| **8** | 8,0 | ohne | 0,02 M Tris | | 0,005% (w/v) Tween 80 | | | + | 23,9 (o) | | - | |
| **9** | 8,0 | ohne | 0,02 M Tris | | 0,5 M L-Arginin-HCl | | | - | n.d. | | - | |
| **10** | 7,5 | ohne | 0,02 M Na-Phosph. | | 10% (w/v) Glyzerin | | | - | n.d. | | - | |

| | | | | | | | | | | | | |
|---|---|---|---|---|---|---|---|---|---|---|---|---|
| ¹ Die *aus "Inclusion Bodies"* isolierten Proteine wurden zunächst mit 6 M Guanidiniumhydrochlorid denaturiert, anschließend 1:50 in einer nicht-denaturierenden Lösung (Lsg. 1-10) verdünnt und über Nacht bei 4°C gehalten. Am Folgetag erfolgte eine organoleptische Kontrolle (OK) bezüglich einer Trübung durch sichtbare Makro-Aggregate bzw. Präzipitate. (-) Trübung; (+) klare Lösung. ² UV/Vis-Spektralanalyse zum Nachweis von unlöslichen Mikro-Aggregaten in klaren Lösungen durch Bestimmung des Verhältnisses der Absorption bei 280 und 330 nm. Test der Ansätze nach Zentrifugation. A₂₈₀/A₃₃₀ ≤ 20: Präzipitation (-); A₂₈₀/A₃₃₀ ≤ 30: Präzipitationstendenz (o); A₂₈o/A₃₃₀ > 30: keine Präzipitation (+). (n.d.) nicht durchgeführt. ³ Bewertung des Löslichkeitsverhaltens auf Basis der organoleptischen und spektrophotometrischen Analyse. (-) tendenziell unlöslich; (+) löslich. | | | | | | | | | | | | |

**Tab. 5: Löslichkeitsverhalten von MPV.7**

| d[P57, 58] | | d[P85] | d[P117] | d[146,155] | d[P180] | d[P211] | P211L | | d[P229] | | d[P256] | K61E, E205K |
|---|---|---|---|---|---|---|---|---|---|---|---|---|
| x | | | x | | x | | x | | x | | | |
| | | | | | | | | | | | | |
| **Lsg.** | **pH** | **NaCl** | **Puffer substanz** | | **Additiv** | | | **OK¹** | | **A₂₈₀/A₃₃₀²** | | **Bewertung³** |
| **1** | 4,5 | 0,15 M | 0,02 M Na-Acetat | | ohne | | | - | | n.d. | | - |
| **2** | 5,5 | 0,15 M | 0,02 M Na-Citrat | | ohne | | | - | | n.d. | | - |
| **3** | 6,5 | 0,15 M | 0,02 M Ka-Phosph. | | ohne | | | - | | n.d. | | - |
| **4** | 7,5 | 0,15 M | 0,02 M Na-Phosph. | | ohne | | | - | | n.d. | | - |
| **5** | 8,0 | 0,15 M | 0,02 M Tris | | ohne | | | + | | 32,5 (+) | | + |
| **6** | 9,0 | 0,15 M | 0,02 M Tris | | ohne | | | + | | 72,5 (+) | | + |
| **7** | 8,0 | 0,075 M | 0,02 M Tris | | 0,5 M L-Arginin-HCl; | | | | | | | |
| | | | | | 0,005% (w/v) Tween | | | + | | 37,6 (+) | | + |
| **8** | 8,0 | ohne | 0,02 M Tris | | 0,005% (w/v) Tween 80 | | | - | | n.d. | | - |
| **9** | 8,0 | ohne | 0,02 M Tris | | 0,5 M L-Arginin-HCl | | | + | | 30,4 (+) | | + |
| **10** | 7,5 | ohne | 0,02 M Na-Phosph. | | 10% (w/v) Glyzerin | | | + | | 43,0 (+) | | + |

| | | | | | | | | | | | | |
|---|---|---|---|---|---|---|---|---|---|---|---|---|
| ¹ Die *aus "Inclusion Bodies"* isolierten Proteine wurden zunächst mit 6 M Guanidiniumhydrochlorid denaturiert, anschließend 1:50 in einer nicht-denaturierenden Lösung (Lsg. 1-10) verdünnt und über Nacht bei 4°C gehalten. Am Folgetag erfolgte eine organoleptische Kontrolle (OK) bezüglich einer Trübung durch sichtbare Makro-Aggregate bzw. Präzipitate. (-) Trübung; (+) klare Lösung. ² UV/Vis-Spektratanatyse zum Nachweis von unlöslichen Mikro-Aggregaten in klaren Lösungen durch Bestimmung des Verhältnisses der Absorption bei 280 und 330 nm. Test der Ansätze nach Zentrifugation. A₂₈₀/A₃₃₀ ≤ 20: Präzipitation (-); A₂₈₀/A₃₃₀ ≤ 30: Präzipitationstendenz (o); A₂₈o/A₃₃₀ > 30: keine Präzipitation (+). (n.d.) nicht durchgeführt. ³ Bewertung des Löslichkeitsverhaltens auf Basis der organoleptischen und spektrophotometrischen Analyse. (-) tendenziell unlöslich; (+) löslich. | | | | | | | | | | | | |

**Tab. 6: Ergebnisse der Molekulargewichts-Analytik von MPV.4, MPV.5, MPV.6 und MPV.7 im Vergleich zu rPhl p 5a wt**

| | | **SEC-MALS-RI²** | | **Beurteilung** | |
|---|---|---|---|---|---|
| **Phl p 5a-Variante** | **MW_{calc.} [kD]¹** | **Peak** | **MW [kD]** | | |
| **Wildtyp** | 28,3 | 1 | 27,3 | Nur Monomere nachweisbar | |
| **MPV.4** | 27,6 | 1 | 28,2-49,3* | Monomer und Dimere in Mischpeak nachweisbar | |
| **MPV.5** | 27,8 | 1 | 27,2 | Nur Monomere nachweisbar | |
| **MPV.6** | 27,6 | 1 | 53,0 | Dimer-Peak | Monomere und Dimere nachweisbar |
| | | 2 | 29,8 | Monomer-Peak | |
| **MPV.7** | 27,8 | 1 | 27,2 | Nur Monomere nachweisbar | |

| | | | | | |
|---|---|---|---|---|---|
| ¹ Kalkuliertes Molekulargewicht (MW_{calc.}) ohne Start-Methionin auf Grundlage der Aminosäuresequenz (Software: DNA-Star, Lasergene, USA) ² Bestimmung der Partikelmasse durch SEC-MALS. Angegeben ist der Durchschnitt der Masse der eluierten Proteinpartikel im gesetzten Peakfenster mit Ausnahme der Messung von MPV.4 (*), bei der der Streubereich der Massen in dem Gesamtpeak angegeben ist. | | | | | |

Zur Online-Bestimmung der Proteinkonzentration wurde der Brechungsindex-Detektor (RI) OptilabrEX (Wyatt, Santa Barbara, USA) eingesetzt. Die Lichtstreuung der Partikel wurde mit dem Mehrwinkeldetektor MiniDAWN Treos (Wyatt) bestimmt. Die Berechnung der Partikelmasse erfolgte mit der Software ASTRA 5.3.2.17 (Wyatt) über Debeye-Formalismus mit einem angenommenen Brechungsindexinkrement von 0,180 ml/g.
Säule: Superdex 200 GL 10/ 300 (GE Healthcare, Uppsala, Schweden). Der Größenausschluss (*t*₀) liegt bei 20,45 min (entspricht -670 kD).
Laufmittel: 20 mM Tris 8,0, 150 mM NaCl

**Tab. 7: Nachweis der T-Zellreaktivität von MPV.4**

| | | | **Stimulationsindex¹** | | | |
|---|---|---|---|---|---|---|
| **Donor²** | **T**-**Zell**-**Linie** | | **rPhl p 5a wt** | **MPV.4** | | **Reaktivität von MPV.4 relativ zu rPhl p 5a wt³** |
| **3** | 3.10 | | 3,0 | 4,0 | | 1,33 |
| **8** | 8.2 | | 13 | 12,7 | | 0,98 |
| **8** | 8.3 | | 2,9 | 4,0 | | 1,38 |
| **19** | 19.1 | | 7,2 | 9,9 | | 1,38 |
| **19** | 19.2 | | 3,5 | 4,9 | | 1,40 |
| **21** | 21.210 | | 4,5 | 5,8 | | 1,29 |
| **23** | 23.22 | | 4,3 | 2,2 | | 0,51 |
| **55** | 55.184 | | 26,7 | 28,5 | | 1,07 |
| **55** | 55.193 | | 49,9 | 46,2 | | 0,93 |
| **59** | 59.57 | | 4,1 | 4,4 | | 1,07 |
| **59** | 59.91 | | 7,7 | 9,0 | | 1,17 |
| **60** | 60.162 | | 4,9 | 2,6 | | 0,53 |
| **65** | 65.115 | | 6,7 | 9,1 | | 1,36 |
| **116** | 116.34 | | 9,4 | 2,8 | | 0,30 |
| **128** | 128.40 | | 8,8 | 8,9 | | 1,01 |
| **137** | 137.41 | | 2,8 | 6,5 | | 2,32 |
| **137** | 137.43 | | 2,6 | 2,5 | | 0,96 |
| | | | | | | **1,12 Mittelwert** |
| | | | | | | 0,45 SD |

| | | | | | | |
|---|---|---|---|---|---|---|
| ¹ Stimulationsindex (SI), berechnet von [³H]-Messwerten des Proliferationstests. cpm-Messwerte Allergen-stimulierter Zellkulturen/ cpm-Messwerte unstimulierter Zellkulturen. ² Donor: Klinisch definierter Gräserpollen-Allergiker. ³ Berechnet mit SI (MPV.4)/ SI (rPhl p 5a wt). SD: Standardabweichung. | | | | | | |

**Tab. 8 Nachweis der T-Zellreaktivität von MPV.7**

| | | | **Stimulationsindex¹** | | | |
|---|---|---|---|---|---|---|
| **Donor²** | **T-Zell-Linie** | | **rPhl p 5a wt** | **MPV.7** | | **Reaktivität von MPV.7 relativ zu rPhl p 5a wt³** |
| **3** | 3.10 | | 2,0 | 2,2 | | 1,10 |
| **8** | 8.2 | | 27,7 | 23,6 | | 0,85 |
| **8** | 8.3 | | 5,2 | 8,2 | | 1,58 |
| **11** | 11.2 | | 2,1 | 2,7 | | 1,29 |
| **11** | 11.3 | | 2,4 | 2,4 | | 1,00 |
| **19** | 19.1 | | 4,8 | 6,5 | | 1,35 |
| **21** | 21.2 | | 3,7 | 3,4 | | 0,92 |
| **21** | 21.210 | | 3,9 | 5,4 | | 1,38 |
| **55** | 55.181 | | 12,4 | 14,8 | | 1,19 |
| **55** | 55.184 | | 123,6 | 149,5 | | 1,21 |
| **59** | 59.91 | | 4,5 | 5,0 | | 1,11 |
| **60** | 60.162 | | 2,9 | 2,6 | | 0,90 |
| **65** | 65.115 | | 4,6 | 4,7 | | 1,02 |
| **70** | 70.126 | | 6,5 | 6,5 | | 1,00 |
| **116** | 116.34 | | 8,5 | 2,5 | | 0,29 |
| **128** | 128.40 | | 4,8 | 3,5 | | 0,73 |
| | | | | | | **1**,**06 Mittelwert** |
| | | | | | | 0,30 SD |

| | | | | | | |
|---|---|---|---|---|---|---|
| ¹ Stimulationsindex (SI), berechnet von [³H]-Messwerten des Proliferationstests. cpm-Messwerte Allergen-stimulierter Zellkulturen/ cpm-Messwerte unstimulierter Zellkulturen. ² Donor: Klinisch definierter Gräserpollen-Allergiker. ³ Berechnet mit SI (MPV.7)/ SI (rPhl p 5a wt). SD: Standardabweichung. | | | | | | |

## Patentansprüche

1. Hypoallergene Variante eines Gruppe 5-Allergens der Familie der Süßgräser (Poaceae), bei der die Proline, die in einem Alignment den Prolinen der Positionen 57, 58, 117, 146, 155, 180, 211 in der Aminosäuresequenz des Wildtyp-Phl p 5.0109 entsprechen, einzeln oder in Kombinationen mutiert sind.

2. Hypoallergene Variante gemäß Anspruch 1, **dadurch gekennzeichnet, dass** das Prolin, das in einem Alignment dem Prolin 211 des Wildtyp-Phl p 5.0109 entspricht, nicht deletiert, sondern durch eine andere Aminosäure ersetzt wird.

3. Hypoallergene Variante gemäß Anspruch 1 oder 2, bei der die Proline, die in einem Alignment den Prolinen der Positionen 146 und 155 in der Aminosäuresequenz des Wildtyp-Phl p 5.0109 entsprechen, nicht mutiert werden.

4. Hypoallergene Variante gemäß einem oder mehreren der Ansprüche 1 bis 3, bei der zusätzlich die Aminosäuren, die in einem Alignment Lysin der Position 61 und Glutaminsäure der Position 205 in der Aminosäuresequenz des Wildtyp-Phl p 5.0109 entsprechen, einzeln oder in Kombinationen mutiert sind.

5. Hypoallergene Variante gemäß einem oder mehreren der Ansprüche 1 bis 4, **dadurch gekennzeichnet, dass** es sich bei dem Gruppe 5-Allergen um ein Gruppe 5-Allergen der Unterfamilie Pooideae handelt.

6. Hypoallergene Variante gemäß einem oder mehreren der Ansprüche 1 bis 5, **dadurch gekennzeichnet, dass** das Gruppe 5-Allergen ausgewählt ist aus der Gruppe bestehend aus Phl p 5, Lol p 5, Poa p 5, Hol I 5, Dac g 5, Pha a 5, Ant o 5, Fes p 5, Hor v 5, Sec c 5 und Tri a 5.

7. Hypoallergene Variante gemäß einem oder mehreren der Ansprüche 1 bis 6, **dadurch gekennzeichnet, dass** es sich um ein Fragment oder eine Variante einer hypoallergenen Variante gemäß einem oder mehreren der Ansprüche 1 bis 6 oder um ein Multimer einer oder mehrerer hypoallergener Varianten gemäß einem oder mehreren der Ansprüche 1 bis 6 handelt oder **dadurch gekennzeichnet, dass** eine oder mehrere hypoallergene Varianten gemäß einem oder mehreren der Ansprüche 1 bis 6 oder deren Fragmente, Varianten oder Multimere Bestandteil eines rekombinanten Fusionsproteins sind.

8. DNA-Molekül, das für eine hypoallergenen Variante gemäß einem oder mehreren der Ansprüche 1 bis 7 kodiert.

9. Rekombinanter Expressionsvektor, enthaltend ein DNA-Molekül gemäß Anspruch 8, funktionell verbunden mit einer Expressions-Kontrollsequenz.

10. Nicht-menschlicher Wirtsorganismus, transformiert mit einem DNA-Molekül gemäß Anspruch 8 oder einem rekombinanten Expressionsvektor gemäß Anspruch 9.

11. Verfahren zur Herstellung einer hypoallergenen Variante gemäß einem oder mehrerer der Ansprüche 1 bis 7 durch Kultivierung eines nichtmenschlichen Wirtsorganismus gemäß Anspruch 10 und Gewinnung der entsprechenden Allergenvariante aus der Kultur.

12. Hypoallergene Variante gemäß einem oder mehrerer der Ansprüche 1 bis 7 als Arzneimittel.

13. DNA-Molekül gemäß Anspruch 8 als Arzneimittel.

14. Rekombinanter Expressionsvektor gemäß Anspruch 9 als Arzneimittel.

15. Verwendung wenigstens einer hypoallergenen Variante gemäß einem oder mehreren der Ansprüche 1 bis 7 zur Herstellung eines Arzneimittels zur Prävention und/oder therapeutischen Behandlung von Typ-1 Allergien, an deren Auslösung Gruppe 5-Allergene der Süßgräser ursächlich beteiligt sind.

16. Verwendung wenigstens eines DNA-Moleküls gemäß Anspruch 8 und/oder eines rekombinanten Expressionsvektors gemäß Anspruch 9 zur Herstellung eines Arzneimittels zur immuntherapeutischen DNA-Vakzinierung.

17. Pharmazeutische Zubereitung, enthaltend wenigstens eine hypoallergene Variante gemäß einem oder mehreren der Ansprüche 1 bis 7, wenigstens ein DNA-Molekül gemäß Anspruch 8 und/oder wenigstens einen rekombinanten Expressionsvektor gemäß Anspruch 9 und gegebenenfalls weitere Wirk- und/oder Hilfsstoffe zur Prävention und/oder therapeutischen Behandlung von Typ-1 Allergien.

18. Pharmazeutische Zubereitung gemäß Anspruch 17, **dadurch gekennzeichnet, dass** es sich bei den weiteren Wirkstoffen um Allergene der Süßgräser oder Varianten davon handelt.
